(19) Europäisches Patentamt
European Patent Office
Office européen des brevets

(11) EP 0 754 709 A1

(12) **EUROPÄISCHE PATENTANMELDUNG**

(43) Veröffentlichungstag:
**22.01.1997 Patentblatt 1997/04**

(51) Int. Cl.[6]: **C08F 220/34**, C08F 220/18, G02F 1/35

(21) Anmeldenummer: **96110715.8**

(22) Anmeldetag: **03.07.1996**

(84) Benannte Vertragsstaaten:
**DE FR NL**

(30) Priorität: **12.07.1995 DE 19525304**

(71) Anmelder: **BASF AKTIENGESELLSCHAFT
67056 Ludwigshafen (DE)**

(72) Erfinder:
- **Beckmann, Stefan, Dr.
67098 Bad Dürkheim (DE)**
- **Etzbach, Karl-Heinz, Dr.
67227 Frankenthal (DE)**
- **Sens, Rüdiger, Dr.
68165 Mannheim (DE)**

(54) **Copolymere mit Adamantyl- und Farbstoffseitenketten**

(57) Adamantylgruppen tragende Copolymere aus

I) 1 bis 99 mol-% eines Monomeren der allgemeinen Formel I

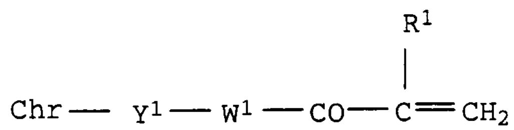

I

II) 1 bis 99 mol-% eines Monomeren der allgemeinen Formel II

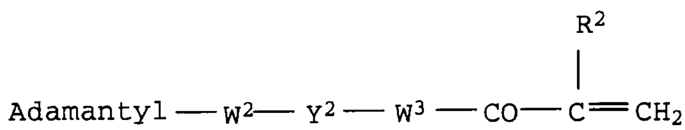

II

und

III) 0 bis 90 mol-% weiterer radikalisch polymerisierbarer Monomerer (III),

mit

| | |
|---|---|
| Chr = | Farbstoff mit -CH=, -N=, -N=N-, -CH=N- oder $-CH_2=CH_2$-Struktur, |
| $R^1$,$R^2$ = | H, D, $CH_3$, $CD_3$, Cl, |
| $Y^1$,$Y^2$ = | $C_2$-$C_{11}$-Alkylen, gegebenenfalls durch O, NH oder N($C_1$-$C_4$-Alkyl) unterbrochen oder chemische Bindung |
| $W^1$,$W^2$,$W^3$ = | O, NH, N($C_1$-$C_4$-Alkyl) |

sowie Verfahren zu deren Herstellung. Die Copolymere finden Verwendung in der nichtlinearen Optik.

EP 0 754 709 A1

Printed by Rank Xerox (UK) Business Services
2.13.13/3.4

## Beschreibung

Die vorliegende Erfindung betrifft Adamantylgruppen tragende Copolymere aus

I) 1 bis 99 mol-% eines Monomeren der allgemeinen Formel I

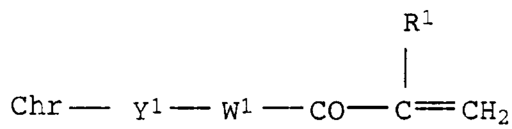

I

II) 1 bis 99 mol-% eines Monomeren der allgemeinen Formel II

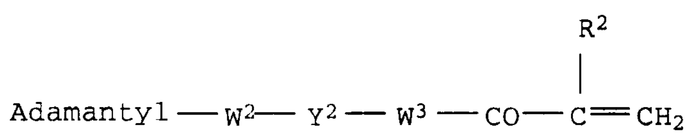

II

und

III) 0 bis 90 mol-% weiterer radikalisch polymerisierbarer Monomerer (III),

wobei die Substituenten und Brückenglieder die folgende Bedeutung haben:

Chr der Rest eines Farbstoffes aus der Methin-, Azamethin-, Azo-, Methylenamino- oder Vinylenreihe, wobei im Falle der Azo-, Methylenamino- und Vinylenfarbstoffreste mindestens einer der aromatischen Ringe des Chromophors mindestens ein Heteroatom enthält,

$R^1$,$R^2$ Wasserstoff, Deuterium, Methyl, trideuteriertes Methyl oder Chlor,

$Y^1$,$Y^2$ $C_2$-$C_{11}$-Alkylen oder durch bis zu 4 Sauerstoffatome in Etherfunktion oder durch nicht benachbarte Imino- oder $C_1$-$C_4$-Alkyliminogruppen unterbrochenes $C_2$-$C_{10}$-Alkylen oder eine chemische Bindung,

$W^1$,$W^2$,$W^3$ Sauerstoff, Imino oder $C_1$-$C_4$-Alkylimino, wobei für den Fall, daß $Y^1$ oder $Y^2$ eine chemische Bindung ist, $W^1$ bzw. $W^2$ entfällt.

Aus J. Polymer Sci. Part A, Polymer Chem., Band 28, Seiten 1-13, 1990 sowie aus EP-A 535 490 sind Polymerisate bekannt, die in den Seitenketten Azofarbstoffe als Chromophore enthalten.

Aus EP-A 572 898 sind Methin- und Azamethinfarbstoffe tragende Polymerisate mit nichtlinear optischen (=NLO)Eigenschaften bekannt.

Aus der EP-A 590 421 sind Copolymerisate mit nichtlinear optischen Eigenschaften bekannt, die neben Farbstoffseitenketten auch Adamantylreste enthalten.

Um aus diesen Polymeren NLO-aktive Filme herzustellen, müssen die Seitengruppen im elektrischen Feld orientiert werden. Dies geschieht üblicherweise im Bereich der Glastemperatur, bei der die Seitengruppen sehr beweglich sind. Durch Abkühlung kann die im elektrischen Feld erzielte Orientierung eingefroren werden. Ein Nachteil der bekannten Polymeren besteht in der zu schnellen Relaxation der orientierten Chromophorseitengruppen, d.h. einem Verlust der Orientierung.

Aufgabe der vorliegenden Erfindung war es, neue Copolymerisate bereitzustellen, die eine hohe Glasübergangstemperatur und damit verbunden eine verbesserte Relaxationsstabilität sowie eine hohe Suszeptibilität 2. Ordnung, d.h. ausgeprägte nichtlinear optische Eigenschaften aufweisen.

Demgemäß wurden die oben bezeichneten chromophorhaltigen Adamantylcopolymere gefunden.

Geeignete Farbstoffe in diesen Copolymeren sind beispielsweise solche der Formel Ia

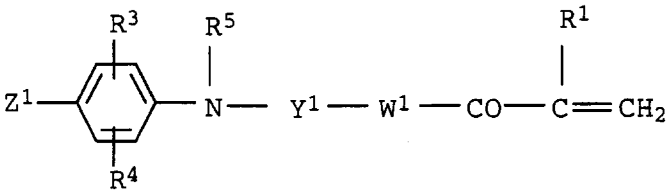

Ia

wobei

- $R^3$,$R^4$ Wasserstoff, $C_1$-$C_6$-Alkyl, $C_1$-$C_6$-Alkoxy, durch Phenyl oder $C_1$-$C_4$-Alkoxy substituiertes $C_1$-$C_6$-Alkoxy,
- $R^5$ Wasserstoff, $C_1$-$C_6$-Alkyl, ein Cycloalkylrest mit 5 - 7 C-Atomen im Ring oder $C_3$-$C_4$-Alkenyl und
- $Z^1$ eine der folgenden Gruppen
  D-N=N-
  D-CH=N-
  D-N=CH-
  D-CH=CH-
  G=CH-
  G=CH-CH=CH-
  G=N-
  bedeuten, in der
- D für einen fünfgliedrigen heteroaromatischen Rest mit bis zu drei der Heteroatome Stickstoff, Sauerstoff und/oder Schwefel steht, wobei dieser Rest durch einen Benzol-, Thiophen-, Pyridin- oder Pyrimidinring anelliert sein kann und
- G für einen zweiwertigen chinoiden Rest steht, der sich von der Benzol-, Naphthalin-, Pyridin-, Chinolin-, Thiazol-, Benzthiophen-, Triazolopyridin- oder 1,3-Dioxanreihe ableitet.

Von den Farbstoffen der Azoreihe (-N=N-), der Methylenaminoreihe (-N=CH- oder -CH=N-) und der Vinylenreihe (-CH=CH-) sind solche bevorzugt, in denen D ein Rest der Pyrrol-, Furan-, Thiophen-, Pyrazol-, Imidazol-, Oxazol-, Isoxazol-, Thiazol-, Isothiazol-, Triazol-, Oxdiazol-, Thiadiazol-, Benzofuran-, Benzthiophen-, Benzimidazol-, Benzooxazol-, Benzthiazol-, Benzisothiazol-, Pyridothiophen-, Pyrimidinothiophen-, Thienothiophen- oder Thienothiazolreihe ist.

Besonders zu nennen sind solche Reste D, die von Aminoheterocyclen der Formeln

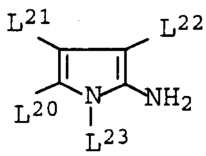
(Va)

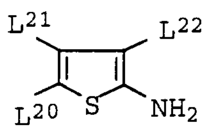
(Vb)

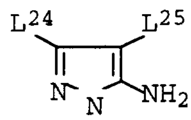
(Vc)

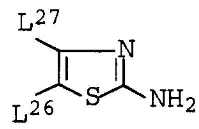
(Vd)

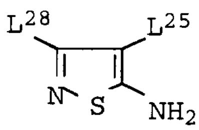
(Ve)

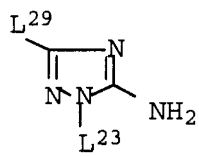
(Vf)

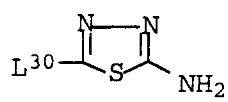
(Vg)

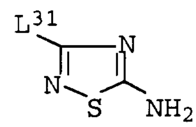
(Vh)

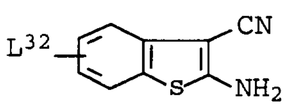
(Vi)

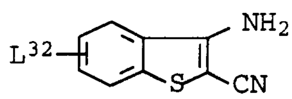
(Vj)

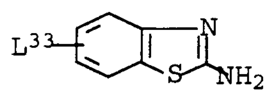
(Vk)

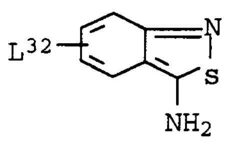
(Vl)

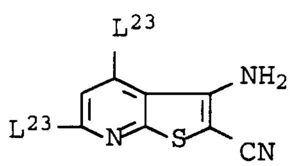
(Vm)

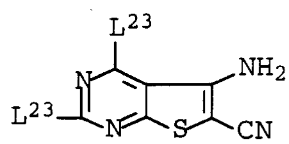
(Vn)

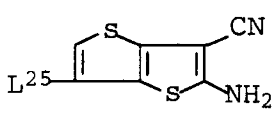
(Vo)

oder

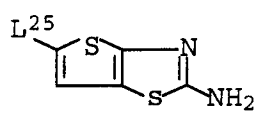
(Vp)

stammen, worin die Substituenten folgende Bedeutung haben:

$L^{20}$ Nitro, Cyano, $C^1$-$C_6$-Alkanoyl, Benzoyl, $C_1$-$C_6$-Alkylsulfonyl, gegebenenfalls substituiertes Phenylsulfonyl oder einen Rest der Formel -CH=T, in der T für den Rest einer CH-aciden Verbindung steht, besonders bevorzugt für $L^{20}$ ist die Gruppe

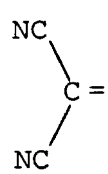

$L^{21}$ Wasserstoff, $C_1$-$C_6$-Alkyl, Phenyl oder durch $C_1$-$C_6$-Alkyl, $C_1$-$C_6$-Alkoxy oder Hydroxy substituiertes Phenyl, Halogen, Hydroxy, Mercapto, gegebenenfalls durch Phenyl oder $C_1$-$C_4$-Alkoxy substituiertes $C_1$-$C_6$-Alkoxy, gegebenenfalls substituiertes Phenoxy, gegebenenfalls durch Phenyl substituiertes $C_1$-$C_6$-Alkylthio, gegebenenfalls substituiertes Phenylthio, $C_1$-$C_6$-Alkylsulfonyl oder gegebenenfalls substituiertes Phenylsulfonyl,

$L^{22}$ Cyano, $C_1$-$C_4$-Alkoxycarbonyl oder Nitro,

$L^{23}$ Wasserstoff, $C_1$-$C_6$-Alkyl oder Phenyl,

$L^{24}$ $C_1$-$C_6$-Alkyl oder Phenyl,

$L^{25}$ Cyano, $C_1$-$C_4$-Alkoxycarbonyl, $C_1$-$C_6$-Alkanoyl oder Halogen, sowie gegebenenfalls mit diesen Resten substituierte Phenylreste,

$L^{26}$ Nitro, Cyano, $C_1$-$C_6$-Alkanoyl, Benzoyl, $C_1$-$C_6$-Alkylsulfonyl, gegebenenfalls substituiertes Phenylsulfonyl oder einen Rest der Formel -CH=T,

$L^{27}$ Wasserstoff, $C_1$-$C_6$-Alkyl, Halogen, gegebenenfalls durch Phenyl oder $C_1$-$C_4$-Alkoxy substituiertes $C_1$-$C_4$-Alkoxy, gegebenenfalls durch Phenyl substituiertes $C_1$-$C_6$-Alkylthio, gegebenenfalls substituiertes Phenylthio, $C_1$-$C_6$-Alkylsulfonyl, gegebenenfalls substituiertes Phenylsulfonyl oder $C_1$-$C_4$-Alkoxycarbonyl,

$L^{28}$ Cyano, gegebenenfalls durch Phenyl substituiertes $C_1$-$C_6$-Alkyl, gegebenenfalls durch Phenyl substituiertes $C_1$-$C_6$-Alkylthio, gegebenenfalls substituiertes Phenyl, Thienyl, $C_1$-$C_4$-Alkylthienyl, Pyridyl oder $C_1$-$C_4$-Alkylpyridyl,

$L^{29}$ Phenyl oder Pyridyl,

$L^{30}$ Trifluormethyl, Nitro, $C_1$-$C_6$-Alkyl, Phenyl, gegebenenfalls durch Phenyl substituiertes $C_1$-$C_8$-Alkylthio oder $C_1$-$C_4$-Dialkylamino,

$L^{31}$ $C_1$-$C_6$-Alkyl, Phenyl, 2-Cyanoethylthio oder 2-($C_1$-$C_4$-Alkoxycarbonyl)ethylthio,

$L^{32}$ Wasserstoff, Nitro oder Halogen und

$L^{33}$ Wasserstoff, Cyano, Nitro oder Halogen bedeuten.

Die Phenylgruppen können ihrerseits auch folgende Substituenten tragen: $C_1$-$C_4$-Alkyl, $C_1$-$C_4$-Alkoxy oder Halogen, dabei insbesondere Chlor oder Brom. Die Phenylringe weisen dabei in der Regel 1 bis 3 Substituenten auf.

Alle in den obengenannten Formeln auftretenden Alkyl-, Alkylen- oder Alkenylgruppen können sowohl geradkettig als auch verzweigt sein.

Reste $R^3$, $R^4$, $R^5$, $L^{21}$, $L^{23}$, $L^{24}$, $L^{27}$, $L^{28}$, $L^{30}$ und $L^{31}$ sind z.B. Methyl, Ethyl, Propyl, Isopropyl, Butyl, Isobutyl, sec-Butyl, Pentyl, Isopentyl, Neopentyl, tert-Pentyl, Hexyl oder 2-Methylpentyl.

Reste $L^{28}$ sind weiterhin z.B. Benzyl oder 1- oder 2-Phenylethyl.

Reste $L^{21}$, $L^{27}$, $L^{28}$ und $L^{30}$ sind weiterhin z.B. Methylthio, Ethylthio, Propylthio, Isopropylthio, Butylthio, Isobutylthio, Pentylthio, Hexylthio, Benzylthio oder 1- oder 2-Phenylethylthio.

Reste $L^{21}$ und $L^{27}$ sind weiterhin z.B. Phenylthio, 2-Methylphenylthio, 2-Methoxyphenylthio oder 2-Chlorphenylthio.

Reste $R^3$, $R^4$, $L^{21}$ und $L^{27}$ sind weiterhin z.B. Methoxy, Ethoxy, Propoxy, Isopropoxy, Butoxy, Isobutoxy, sec-Butoxy, Pentyloxy, Isopentyloxy, Neopentyloxy, tert-Pentyloxy, Hexyloxy oder 2-Methylpentyloxy.

Reste $L^{21}$, $L^{25}$, $L^{27}$, $L^{32}$ und $L^{33}$ sind weiterhin z.B. Fluor, Chlor oder Brom.

Reste $L^{20}$, $L^{21}$, $L^{26}$ und $L^{27}$ sind weiterhin z.B. Methylsulfonyl, Ethylsulfonyl, Propylsulfonyl, Isopropylsulfonyl, Butylsulfonyl, Isobutylsulfonyl, sec-Butylsulfonyl, Pentylsulfonyl, Isopentylsulfonyl, Neopentylsulfonyl, Hexylsulfonyl, Phenylsulfonyl, 2-Methylphenylsulfonyl, 2-Methoxyphenylsulfonyl oder 2-Chlorphenylsulfonyl.

Reste $L^{22}$, $L^{25}$ und $L^{27}$ sind weiterhin z.B. Methoxycarbonyl, Ethoxycarbonyl, Propoxycarbonyl, Isopropoxycarbonyl, Butoxycarbonyl, Isobutoxycarbonyl oder sec-Butoxycarbonyl.

Reste $R^3$, $R^4$, $L^{21}$ und $L^{27}$ sind weiterhin z.B. 2-Methoxyethoxy, 2-Ethoxyethoxy, 2- oder 3-Methoxypropoxy, 2- oder 3-Ethoxypropoxy, 2- oder 4-Methoxybutoxy, 2- oder 4-Ethoxybutoxy, 5-Methoxypentyloxy, 5-Ethoxypentyloxy, 6-Methoxyhexyloxy, 6-Ethoxyhexyloxy, Benzyloxy oder 1- oder 2-Phenylethoxy.

Reste $L^{30}$ sind weiterhin z.B. Dimethylamino, Diethylamino, Dipropylamino, Diisopropylamino, Dibutylamino oder N-Methyl-N-ethylamino.

Reste $L^{31}$ sind weiterhin z.B. 2-Methoxycarbonylethylthio oder 2-Ethoxycarbonylethylthio.

Reste $R^5$ sind weiterhin z.B. Cyclopentyl, Cyclohexyl, Cycloheptyl, Allyl oder Methallyl.

Reste $L^{28}$ sind weiterhin z.B. Phenyl, 2-, 3- oder 4-Methylphenyl, 2,4-Dimethylphenyl, 2-, 3- oder 4-Chlorphenyl, 2-, 3- oder 4-Methoxyphenyl, 2- oder 3-Methylthienyl oder 2-, 3- oder 4-Methylpyridyl.

Reste $Y^1$ sind z.B. $(CH_2)_2$, $(CH_2)_3$, $(CH_2)_4$, $(CH_2)_5$, $(CH_2)_6$, $(CH_2)_7$, $(CH_2)_8$, $(CH_2)_9$, $(CH_2)_{10}$, $CH(CH_3)$-$CH_2$,

$CH(CH_3)$-$CH(CH_3)$, $C_2H_4$-o-$C_2H_4$, $C_2H_4$-NH-$C_2H_4$, $C_2H_4$-$N(CH_3)$-$C_2H_4$, $C_2H_4O$-$C_2H_4$-O-$C_2H_4$, $C_2H_4$-NH-$C_2H_4$-NH-$C_2H_4$ oder $C_2H_4$-$N(CH_3)$-$C_2H_4$-$N(CH_3)$-$C_2H_4$ oder eine chemische Bindung.

Reste $W^1$, $W^2$ und $W^3$ sind z.B. Sauerstoff, Imino, Methylimino, Ethylimino, Propylimino, Isopropylimino oder Butylimino.

Reste $L^{20}$, $L^{25}$ und $L^{26}$ sind weiterhin z.B. Formyl, Acetyl, Propionyl, Butyryl, Pentanoyl oder Hexanoyl.

Wenn $L^{20}$ oder $L^{26}$ für den Rest -CH=T stehen, worin T sich von einer CH-aciden Verbindung $H_2T$ ableitet, kommen als CH-acide Verbindungen $H_2T$ z.B. Verbindungen der Formel

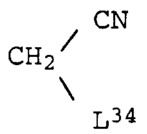 , 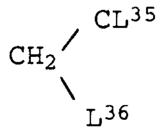 , 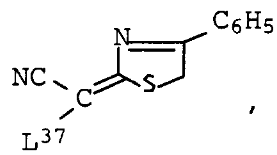 , 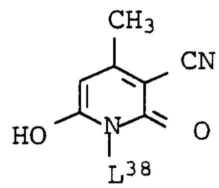

(VIa) (VIb) (VIc)

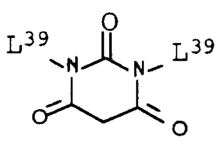 , 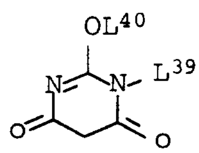 oder 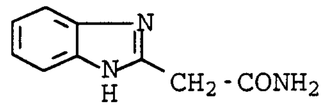

(VIe) (VIf) (VIg)

in Betracht, wobei die Struktur VIa besonders bevorzugt ist und

$L^{34}$ Cyano, Nitro, $C_1$-$C_4$-Alkanoyl, gegebenenfalls substituiertes Benzoyl, $C_1$-$C_4$-Alkylsulfonyl, gegebenenfalls substituiertes Phenylsulfonyl, Carboxyl, $C_1$-$C_4$-Alkoxycarbonyl, $C_3$-$C_4$-Alkenyloxycarbonyl, Phenoxycarbonyl, Carbamoyl, $C_1$-$C_4$-Mono- oder Dialkylcarbamoyl, gegebenenfalls substituiertes Phenylcarbamoyl, gegebenenfalls substituiertes Phenyl, Benzthiazol-2-yl, Benzimidazol-2-yl, 5-Phenyl-1,3,4-thiadiazol-2-yl oder 2-Hydroxychinoxalin-3-yl, besonders bevorzugt Cyano,

$L^{35}$ $C_1$-$C_4$-Alkyl, $C_1$-$C_4$-Alkoxy oder $C_3$-$C_4$-Alkenyloxy,

$L^{36}$ $C_1$-$C_4$-Alkoxycarbonyl, $C_3$-$C_4$-Alkenyloxycarbonyl, Phenylcarbamoyl oder Benzimidazol-2-yl,

$L^{37}$ Cyano, $C_1$-$C_4$-Alkoxycarbonyl oder $C_3$-$C_4$-Alkenyloxycarbonyl,

$L^{38}$ Wasserstoff oder $C_1$-$C_6$-Alkyl,

$L^{39}$ Wasserstoff, $C_1$-$C_4$-Alkyl oder Phenyl und

$L^{40}$ $C_1$-$C_4$-Alkyl bedeuten.

Dabei ist der Rest der sich von Verbindungen der Formel VIa, VIb oder VIc ableitet, worin $L^{34}$ Cyano, $C_1$-$C_4$-Alkanoyl, $c_1$-$c_4$- Alkoxycarbonyl oder $C_3$-$C_4$-Alkenyloxycarbonyl, $L^{35}$ $C_1$-$C_4$-Alkyl, $C_1$-$C_4$-Alkoxy oder $C_3$-$C_4$-Alkenyloxy, L36 $C_1$-$C_4$-Alkoxycarbonyl oder $C_3$-$C_4$-Alkenyloxycarbonyl und $L^{37}$ Cyano bedeuten, hervorzuheben.

Besonders hervorzuheben ist dabei der Rest der sich von Verbindungen der Formel VIa, VIb oder VIc ableitet, worin $L^{34}$ Cyano, $C_1$-$C_4$-Alkoxycarbonyl oder $C_3$-$C_4$-Alkenyloxycarbonyl, $L^{35}$ $C_1$-$C_4$-Alkoxy oder $C_2$-$C_4$-Alkenyloxy, $L^{36}$ $C_1$-$C_4$-Alkoxycarbonyl oder $C_3$-$c_4$-Alkenyloxycarbonyl und $L^{37}$ Cyano bedeuten.

Besonders bevorzugt sind Copolymerisate, die als farbstoffhaltiges Monomeres einen Azofarbstoff der folgenden Struktur enthalten:

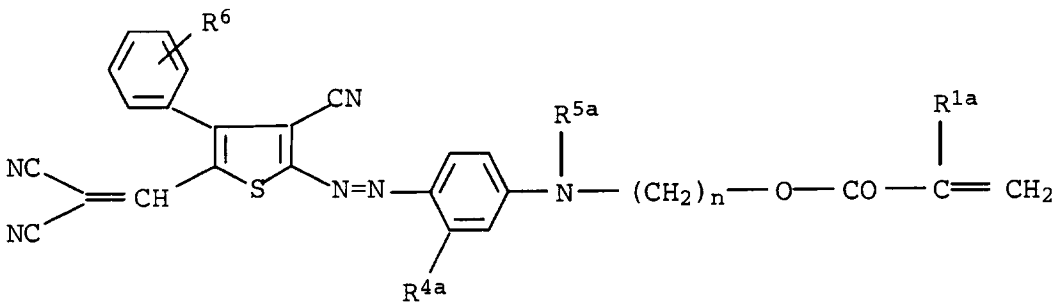

in der

| | |
|---|---|
| $R^6$ | Wasserstoff, $C_1$-$C_6$-Alkyl, $C_1$-$C_6$-Alkoxy oder Hydroxy |
| $R^{4a}$ | Ethyl oder t-Butyl |
| $R^{5a}$ | Ethyl oder Butyl |
| $R^{1a}$ | Wasserstoff oder Methyl und |
| n | 2 bis 6 bedeutet. |

Bevorzugt sind dabei die Polymerisate, in denen

| | |
|---|---|
| $R^6$ | Wasserstoff, Methyl, Ethyl, Methoxy oder Ethoxy |
| $R^{4a}$ | Ethyl oder t-Butyl |
| $R^{5a}$ | Ethyl |
| $R^{1a}$ | Wasserstoff oder Methyl und |
| n | 2,3 oder 6 bedeutet. |

Von den Farbstoffen der Methinreihe (=CH- oder =CH-CH=CH-) oder Azamethinreihe (=N-) sind solche bevorzugt, in denen G einen Rest der Formel

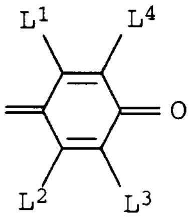 ,

(IVa)

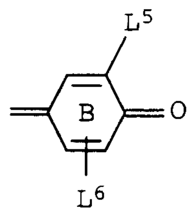 ,

(IVb)

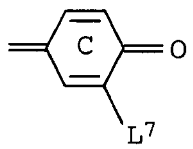 ,

(IVc)

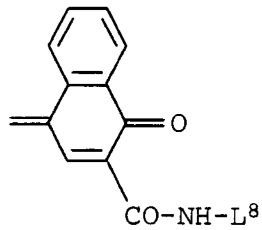 ,

(IVd)

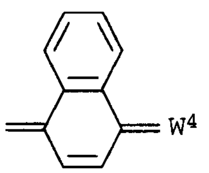 ,

(IVe)

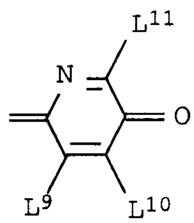 ,

(IVf)

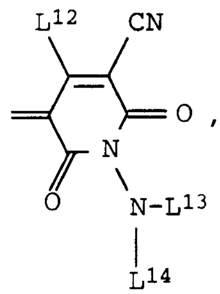 ,

(IVg)

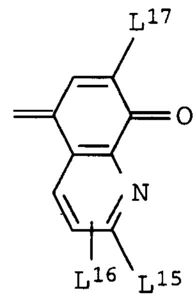 ,

(IVh)

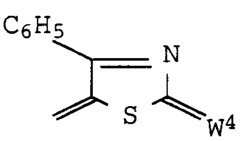 ,

(IVi)

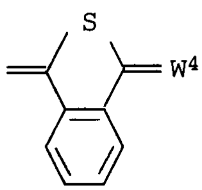 ,

(IVj)

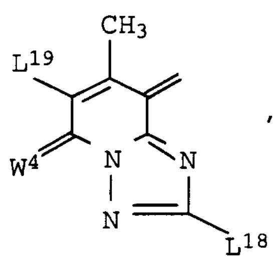 ,

(IVk)

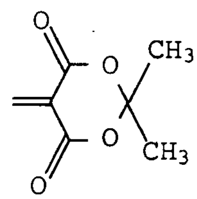

(IVl)

bedeutet, worin
die Ringe B und C benzoanelliert sein können und

$L^1$, $L^2$ und $L^3$ unabhängig voneinander jeweils für Wasserstoff, Methyl, Fluor oder Chlor,

$L^4$ für Fluor oder Chlor,

$L^5$ für Wasserstoff, Fluor, Chlor, Brom, Hydroxy, Nitro, $C_1$-$C_6$-Alkyl, Cyano, $C_1$-$C_4$-Alkylsulfonylamino, $C_1$-$C_6$-Alkoxycarbonyl, $C_1$-$C_4$-Alkylcarbamoyl oder $C_2$-$C_4$-Alkanoylamino,

$L^6$ für Wasserstoff, Fluor, Chlor, Cyano, $C_1$-$C_4$-Alkylcarbamoyl, $C_2$-$C_4$-Alkanoylamino, $C_1$-$C_4$-Alkylureido, $C_1$-$C_4$-Alkylsulfamoyl oder $C_1$-$C_6$-Alkoxycarbonyl,

$L^7$ für einen Rest der Formel

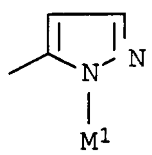 , 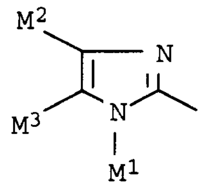 , 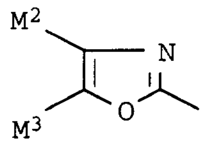 , 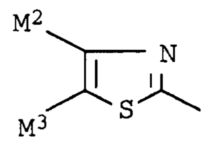 ,

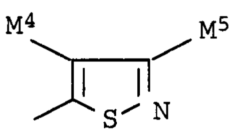 , 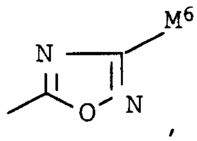 , 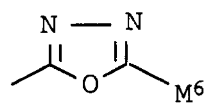 , 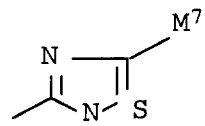

oder 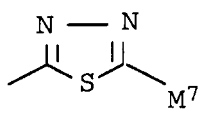 ,

worin

$M^1$ die Bedeutung von Wasserstoff, $C_1$-$C_6$-Alkyl, Benzyl, Cyclohexyl, Phenyl oder Tolyl,

$M^2$ die Bedeutung von Wasserstoff, Chlor, $C_1$-$C_6$-Alkyl, das gegebenenfalls durch ein oder zwei Sauerstoffatome in Etherfunktion unterbrochen ist, Phenyl, $C_1$-$C_6$-Alkoxy, Cyano oder $C_1$-$C_6$-Alkoxycarbonyl, wobei die Alkylgruppe durch ein oder zwei Sauerstoffatome in Etherfunktion unterbrochen sein kann,

$M^3$ die Bedeutung von Wasserstoff, $C_1$-$C_6$-Alkyl, Phenyl, Cyano, Nitro oder $C_1$-$C_6$-Alkoxycarbonyl, wobei die Alkylgruppe durch ein oder zwei Sauerstoffatome in Etherfunktion unterbrochen sein kann, oder $M^2$ und $M^3$ zusammen die Bedeutung eines anellierten Benzorings,

$M^4$ die Bedeutung von Wasserstoff, Chlor, Cyano, Nitro oder $C_1$-$C_6$-Alkoxycarbonyl, wobei die Alkylgruppe durch ein oder zwei Sauerstoffatome in Etherfunktion unterbrochen sein kann,

$M^5$ die Bedeutung von Chlor, $C_1$-$C_6$-Alkyl, das gegebenenfalls durch ein oder zwei Sauerstoffatome in Etherfunktion unterbrochen ist, $C_1$-$C_6$-Alkoxy oder $C_1$-$C_6$-Alkylthio,

$M^6$ die Bedeutung von $C_1$-$C_6$-Alkyl und

$M^7$ die Bedeutung von Wasserstoff, Chlor, Cyano, Thiocyanato, $C_1$-$C_6$-Alkyl, das gegebenenfalls durch ein oder zwei Sauerstoffatome in Etherfunktion unterbrochen ist, $C_1$-$C_6$-Alkoxy, $C_1$-$C_6$-Alkylthio, 2-($C_1$-$C_2$-Alkoxycarbonyl)-ethylthio oder $C_1$-$C_6$-Alkoxycarbonyl, wobei die Alkylgruppe durch ein oder zwei Sauerstoffatome in Etherfunktion unterbrochen sein kann, besitzen,

$L^8$ für einen Rest der Formel

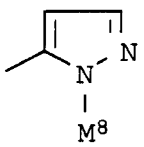 , 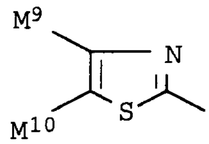 , 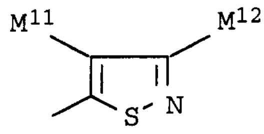 , 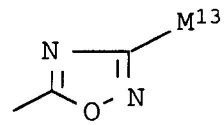 ,

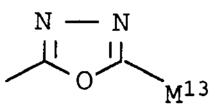 , 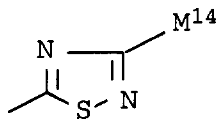 oder 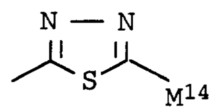 ,

worin

$M^8$ die Bedeutung von $C_1$-$C_6$-Alkyl oder Cyclohexyl,

$M^9$ die Bedeutung von Wasserstoff, Chlor, $C_1$-$C_6$-Alkyl, das gegebenenfalls durch 1 oder 2 Sauerstoffatome in Etherfunktion unterbrochen ist, $C_1$-$C_6$-Alkoxy oder $C_1$-$C_6$-Alkoxycarbonyl, wobei die Alkylgruppe durch 1 oder 2 Sauerstoffatome in Etherfunktion unterbrochen sein kann,

$M^{10}$ die Bedeutung von Wasserstoff, $C_1$-$C_6$-Alkyl, Cyano, Nitro oder $C_1$-$C_6$-Alkoxycarbonyl, wobei die Alkylgruppe durch 1 oder 2 Sauerstoffatome in Etherfunktion unterbrochen sein kann,

$M^{11}$ die Bedeutung von Wasserstoff, Chlor, Cyano, Nitro oder $C_1$-$C_6$-Alkoxycarbonyl, wobei die Alkylgruppe durch 1 oder 2 Sauerstoffatome in Etherfunktion unterbrochen sein kann,

$M^{12}$ die Bedeutung von Chlor, $C_1$-$C_6$-Alkyl, das gegebenenfalls durch 1 oder 2 Sauerstoffatome in Etherfunktion unterbrochen ist, $C_1$-$C_6$-Alkoxy oder $C_1$-$C_6$-Alkylthio,

$M^{13}$ die Bedeutung von $C_1$-$C_6$-Alkyl und

$M^{14}$ die Bedeutung von Wasserstoff, Chlor, Cyano, Thiocyanato, $C_1$-$C_6$-Alkyl, das gegebenenfalls durch 1 oder 2 Sauerstoffatome in Etherfunktion unterbrochen ist, $C_1$-$C_6$-Alkoxy, $C_1$-$C_6$-Alkylthio, 2-($C_1$-$C_2$-Alkoxycarbonyl)ethylthio oder $C_1$-$C_6$-Alkoxycarbonyl, wobei die Alkylgruppe durch 1 oder 2 Sauerstoffatome in Etherfunktion unterbrochen sein kann, besitzen,

$L^9$ für Wasserstoff, Fluor, Chlor, Methyl oder einen Rest der Formel

-NH-CO-$B^1$, -NH-CO-O$B^1$, -NH-CO-N$B^1B^2$, NH-CS-O$B^1$, -NH-CS-N$B^1B^2$, -NH-$SO_2$-$B^1$ oder -NH-$SO_2$-N$B^1B^2$,

worin
$B^1$ und $B^2$ jeweils die Bedeutung von $C_1$-$C_4$-Alkyl besitzen,

$L^{10}$ Wasserstoff, Fluor oder Chlor oder $L^9$ und $L^{10}$ einen anellierten Benzoring,

$L^{11}$ für einen Rest der Formel

-CO-O$B^1$, -CO-NH$B^1$, -CO-NH-CO-$B^1$, -NH-CO-$B^1$, -NH-CO-O$B^1$, -NH-CO-N$B^1B^2$, -NH-CS-O$B^1$, -NH-CS-N$B^1B^2$, -NH-$SO_2$-$B^1$ oder -NH-$SO_2$-N$B^1B^2$,

$L^{12}$ für Wasserstoff oder $C_1$-$C_4$-Alkyl,

$L^{13}$ und $L^{14}$ unabhängig voneinander jeweils für Wasserstoff, $C_1$-$C_{12}$-Alkyl, $C_5$-$C_7$-Cycloalkyl, Phenyl, Pyridyl, $C_1$-$C_6$-Alkanoyl, $C_1$-$C_6$-Alkoxycarbonyl, $C_1$-$C_6$-Alkylsulfonyl, $C_5$-$C_7$-Cycloalkylsulfonyl, Phenylsulfonyl, Pyridylsulfonyl, Benzoyl, Pyridylcarbonyl oder Thienylcarbonyl oder $L^{13}$ und $L^{14}$ zusammen mit dem sie verbindenden Stickstoffatom einen fünf- oder sechsgliedrigen gesättigten heterocyclischen Rest, der gegebenenfalls weitere Heteroatome enthält,

$L^{15}$ für Wasserstoff oder $C_1$-$C_6$-Alkyl,

$L^{16}$ für Wasserstoff, Fluor, Chlor oder Brom,

$L^{17}$ für Fluor, Chlor oder Brom,

$L^{18}$ für $C_1$-$C_{12}$-Alkyl, das durch ein bis drei Sauerstoffatome in Etherfunktion unterbrochen sein kann, Phenyl oder Hydroxy,

| | |
|---|---|
| $L^{19}$ | für Cyano, Carbamoyl, Carboxyl oder $C_1$-$C_6$-Alkoxycarbonyl, |
| $L^{19a}$ | für $C_1$-$C_6$-Alkyl oder $C_2$-$C_4$-Alkenyl und |
| $W^4$ | für Sauerstoff oder einen Rest der Formel |

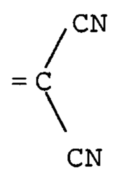 , 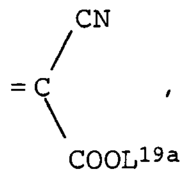 , 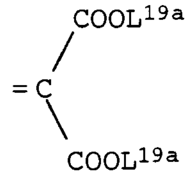

stehen.

Alle in den obengenannten Formeln auftretenden Alkyl-, Alkylen- oder Alkenylgruppen können sowohl geradkettig als auch verzweigt sein.

Reste $L^5$, $L^{13}$, $L^{14}$, $L^{15}$, $L^{18}$, $L^{19a}$, $M^1$, $M^2$, $M^3$, $M^5$, $M^6$, $M^7$, $M^8$, $M^9$, $M^{10}$, $M^{12}$, $M^{13}$, $M^{14}$, $B^1$ und $B^2$ sind z.B. Methyl, Ethyl, Propyl, Isopropyl, Butyl, Isobutyl, sec-Butyl, Pentyl, Isopentyl, Neopentyl, tert-Pentyl, Hexyl oder 2-Methylpentyl.

Reste $L^{13}$, $L^{14}$ und $L^{18}$ sind z.B. Heptyl, Octyl, 2-Ethylhexyl, Isooctyl, Nonyl, Isononyl, Decyl, Isodecyl, Undecyl oder Dodecyl (die obigen Bezeichnungen Isooctyl, Isononyl und Isodecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436).

Reste $M^6$, $M^7$, $M^{12}$ und $M^{14}$ sind z.B. Methylthio, Ethylthio, Propylthio, Isopropylthio, Butylthio, Isobutylthio, Pentylthio, Hexylthio.

Reste $M^2$, $M^6$, $M^7$, $M^9$, $M^{12}$ und $M^{14}$ sind z.B. Methoxy, Ethoxy, Propoxy, Isopropoxy, Butoxy, Isobutoxy, sec-Butoxy, Pentyloxy, Isopentyloxy, Neopentyloxy, tert-Pentyloxy, Hexyloxy oder 2-Methylpentyloxy.

Reste $L^{13}$ und $L^{14}$ sind z.B. Methylsulfonyl, Ethylsulfonyl, Propylsulfonyl, Isopropylsulfonyl, Butylsulfonyl, Isobutylsulfonyl, sec-Butylsulfonyl, Pentylsulfonyl, Isopentylsulfonyl, Neopentylsulfonyl oder Hexylsulfonyl.

Reste $L^5$, $L^6$, $L^{13}$, $L^{14}$, $L^{19}$, $M^2$, $M^3$, $M^4$, $M^9$, $M^{10}$, $M^{11}$ und $M^{14}$ sind z.B. Methoxycarbonyl, Ethoxycarbonyl, Propoxycarbonyl, Isopropoxycarbonyl, Butoxycarbonyl, Isobutoxycarbonyl, sec-Butoxycarbonyl, Pentyloxycarbonyl oder Hexyloxycarbonyl.

Reste $M^2$, $M^3$, $M^4$, $M^7$, $M^9$, $M^{10}$, $M^{11}$ und $M^{14}$ sind z.B. 2-Methoxyethoxycarbonyl, 2-Ethoxyethoxycarbonyl, 3,6-Dioxaheptyloxycarbonyl oder 3,6-Dioxaoctyloxycarbonyl.

Reste $M^7$ und $M^{14}$ sind z.B. 2-Methoxycarbonylethylthio oder 2-Ethoxycarbonylethylthio.

Reste $L^{13}$ und $L^{14}$ sind z.B. Cyclopentyl, Cyclohexyl oder Cycloheptyl.

Reste $L^{13}$ und $L^{14}$ sind z.B. Formyl, Acetyl, Propionyl, Butyryl, Pentanoyl oder Hexanoyl.

Reste $L^5$ sind z.B. Methylsulfonylamino, Ethylsulfonylamino, Propylsulfonylamino, Isopropylsulfonylamino oder Butylsulfonylamino.

Reste $L^5$ und $L^6$ sind z.B. Methylcarbamoyl, Ethylcarbamoyl, Propylcarbamoyl, Isopropylcarbamoyl, Butylcarbamoyl, Acetylamino, Propionylamino oder Butyrylamino.

Reste $L^6$ sind z.B. Methylureido, Ethylureido, Propylureido, Isopropylureido, Butylureido, Methylsulfamoyl, Ethylsulfamoyl, Propylsulfamoyl, Isopropylsulfamoyl oder Butylsulfamoyl.

Reste $M^2$, $M^5$, $M^7$, $M^9$, $M^{12}$, $M^{14}$ und $L^{18}$ sind z.B. 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 4-Methoxybutyl oder 2- oder 4-Ethoxybutyl.

Reste $L^{18}$ sind z.B. 2- oder 3-Butoxypropyl, 2- oder 4-Butoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 4,8-Dioxadecyl, 3,6,8-Trioxadecyl oder 3,6,9-Trioxaundecyl.

Wenn $L^{13}$ und $L^{14}$ zusammen mit dem sie verbindenden Stickstoffatom einen fünf- oder sechsgliedrigen gesättigten heterocyclischen Rest, der gegebenenfalls weitere Heteroatome enthält, bedeuten, so können dafür z.B. Pyrrolidinyl, Piperidinyl, Morpholinyl, Piperazinyl oder N-($C_1$-$C_4$-Alkyl)-piperazinyl in Betracht kommen.

Als weitere geeignete Farbstoffe können die erfindungsgemäßen Copolymerisate solche der allgemeinen Formel Ib enthalten

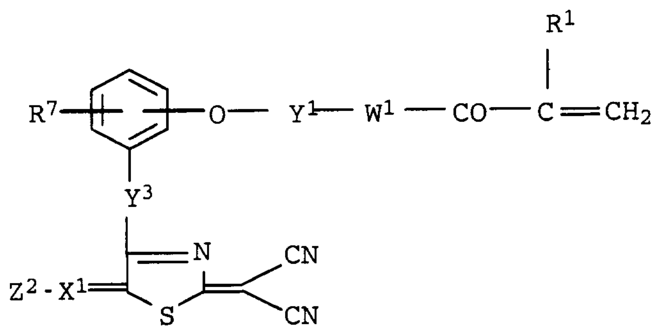

Ib

in der

$Y^3$ eine chemische Bindung, O, S, $SO_2$ oder $NR^5$,

$X^1$ -CH=, -N= oder -CH=CH-CH= und

$Z^2$ sich von einem Ringsystem aus der Benzol-, Naphthalin-, Indol-, Chinolin-, Thiazol- oder Thiophenreihe ableitet und

$R^7$ Wasserstoff, $C_1$-$C_{10}$-Alkyl, $C_3$-$C_8$-Cycloalkyl, $C_1$-$C_{10}$-Alkoxy, OH, $NR^8R^9$, CN, $NO_2$, Halogen oder CHO bedeuten.

$Y^3$ ist bevorzugt Sauerstoff oder eine chemische Bindung, besonders bevorzugt eine chemische Bindung.

$Z^2$ leitet sich bevorzugt von einem Ringsystem aus der Anilin-, Aminonaphthalin-, Aminothiazol oder Aminothiophenreihe ab, besonders bevorzugt aus der Anilinreihe.

Besonders bevorzugt ist $Z^2$ ein Anilinrest der Formel

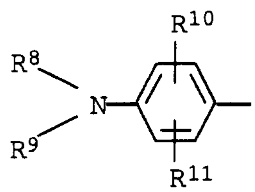

wobei

$R^8$, $R^9$ Wasserstoff, $C_1$-$C_6$-Alkyl, $C_5$-$C_7$-Cycloalkyl, Phenyl oder $C_1$-$C_6$-alkoxysubstituiertes Phenyl, wobei die Reste $R^8$ und $R^9$ auch zu einem Ring geschlossen sein können oder eine vernetzungsfähige Gruppe und

$R^{10}$, $R^{11}$ Wasserstoff, $C_1$-$C_6$-Alkyl, $C_5$-$C_7$-Cycloalkyl, Cyano, Halogen oder $NO_2$ bedeuten.

Weitere besonders geeignete Reste $Z^2$ sind z.B. solche der Formel VIIa bis VIIi

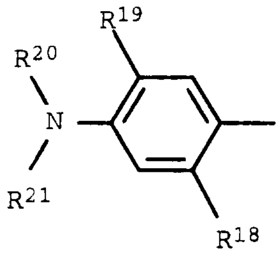 , 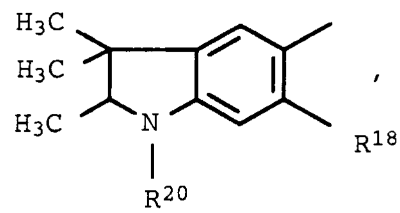 ,

VIIa VIIb

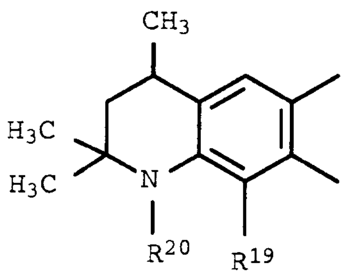 , 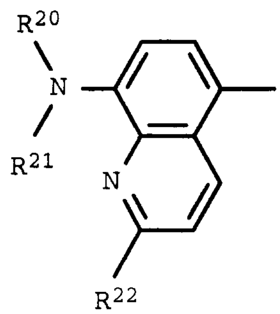 , 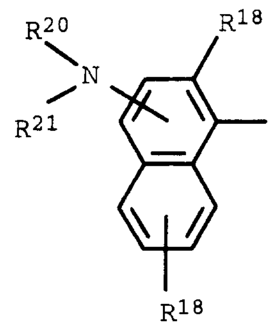 ,

VIIc VIId VIIe

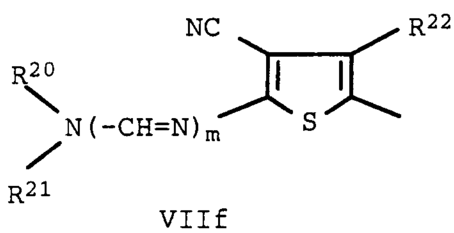 , 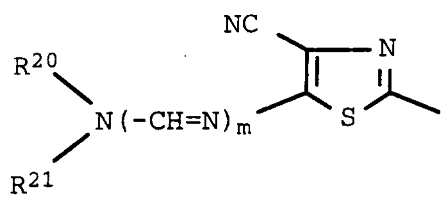 ,

VIIf VIIg

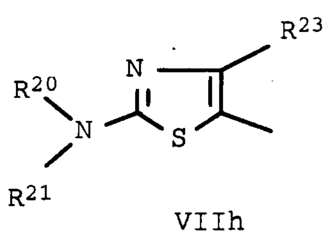 oder 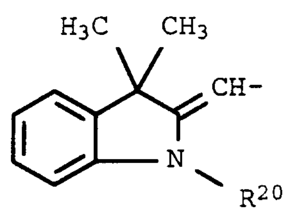 ,

VIIh VIIi

worin

m für 0 oder 1,

$R^{18}$ für Wasserstoff, $C_1$-$C_{10}$-Alkyl, das gegebenenfalls durch 1 oder 2 Sauerstoffatome in Etherfunktion unterbrochen ist, Methoxy, Ethoxy, $C_1$-$C_4$-Alkylsulfonylamino, $C_1$-$C_4$-Mono- oder Dialkylaminosulfonylamino oder den Rest $-NHCOR^{24}$ oder $-NHCO_2R^{24}$, wobei $R^{24}$ die Bedeutung von Phenyl, Benzyl, Tolyl oder $C_1$-$C_8$-Alkyl, das gegebenenfalls durch 1 oder 2 Sauerstoffatome in Etherfunktion unterbrochen ist, besitzt,

$R^{19}$ für Wasserstoff, Methyl, Methoxy oder Ethoxy,

$R^{20}$ und $R^{21}$ gleich oder verschieden sind und jeweils für Wasserstoff, $C_1$-$C_{10}$-Alkyl, das gegebenenfalls substi-

tuiert ist und durch 1 oder 2 Sauerstoffatome in Etherfunktion unterbrochen sein kann, $C_3$-$C_4$-Alkenyl, $C_5$-$C_7$-Cycloalkyl, Phenyl oder Tolyl oder zusammen mit dem sie verbindenden Stickstoffatom für einen 5- oder 6-gliedrigen gesättigten heterocyclischen Rest, der gegebenenfalls weitere Heteroatome enthält,

$R^{22}$ für Halogen oder gegebenenfalls substituiertes Phenyl und

$R^{23}$ für Wasserstoff, Halogen, $C_1$-$C_{10}$-Alkyl, gegebenenfalls substituiertes Phenyl, gegebenenfalls substituiertes Benzyl, Cyclohexyl, Thienyl, Hydroxy oder $C_1$-$C_{10}$-Monoalkylamino stehen.

Alle in den obengenannten Formeln auftretenden Alkyl- und Alkenylgruppen können sowohl geradkettig als auch verzweigt sein.

Wenn in den obengenannten Formeln substituierte Alkylgruppen auftreten, so können als Substituenten, sofern nicht anders vermerkt, z.B. Cyano, Phenyl, Tolyl, Hydroxy, $C_1$-$C_6$-Alkanoyloxy, Acryloyloxy, Methacryloyloxy, $C_1$-$C_4$-Alkoxycarbonyl, $C_1$-$C_4$-Alkylaminocarbonyloxy oder $C_1$-$C_4$-Alkoxycarbonyloxy, wobei im letzten Fall die Alkoxygruppe durch Phenyl oder $C_1$-$C_4$-Alkoxy substituiert sein kann, in Betracht kommen. Sie weisen in der Regel dann 1 oder 2 Substituenten auf.

Wenn in den obengenannten Formeln substituierte Phenylgruppen auftreten, so können als Substituenten z.B. Halogen, $C_1$-$C_4$-Alkyl, Hydroxy oder $C_1$-$C_4$-Alkoxy in Betracht kommen. Sie weisen in der Regel dann 1 bis 3 Substituenten auf.

Geeignete Reste $R^{18}$, $R^{20}$, $R^{21}$, $R^{23}$ und $R^{24}$ sind z.B. Methyl, Ethyl, Propyl, Isopropyl, Butyl, Isobutyl, sec-Butyl, tert-Butyl, Pentyl, Isopentyl, Neopentyl, tert-Pentyl, Hexyl, 2-Methylpentyl, Heptyl, Octyl, 2-Ethylhexyl, Isooctyl, Nonyl, Isononyl, Decyl oder Isodecyl (die Bezeichnungen Isooctyl, Isononyl und Isodecyl sind Trivialbezeichnungen und stammen von den nach der Oxosynthese erhaltenen Alkoholen - vgl. dazu Ullmann's Encyclopedia of Industrial Chemistry, 5th Edition, Vol., A1, Seiten 290 bis 293, sowie Vol. A 10, Seiten 284 und 285).

Reste $R^{18}$, $R^{20}$, $R^{21}$ und $R^{24}$ sind weiterhin z.B. 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Butoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, oder 4,8-Dioxadecyl.

Reste $R^{20}$ und $R^{21}$ sind weiterhin z.B. Benzyl, 2-Methylbenzyl oder 1- oder 2-Phenylethyl.

Reste $R^{23}$ sind weiterhin z.B. Phenyl, 2-, 3- oder 4-Methylphenyl, 2-, 3- oder 4-Ethylphenyl, 2-, 3- oder 4-Propylphenyl, 2-, 3-oder 4-Isopropylphenyl, 2-, 3- oder 4-Butylphenyl, 2,4-Dimethylphenyl, 2-, 3- oder 4-Methoxyphenyl, 2-, 3- oder 4-Ethoxyphenyl, 2-, 3- oder 4-Isobutoxyphenyl, 2,4-Dimethoxyphenyl, 2-, 3- oder 4-Chlorphenyl, 2,4-Dichlorphenyl oder 2-, 3- oder 4-Hydroxyphenyl.

Reste $R^{20}$ und $R^{21}$ sind weiterhin z.B. 2-Cyanoethyl, 2- oder 3-Cyanopropyl, 2-Acetyloxyethyl, 2- oder 3-Acetyloxypropyl, 2-Isobutyryloxyethyl, 2- oder 3-Isobutyryloxypropyl, 2-Methoxycarbonylethyl, 2- oder 3-Methoxycarbonylpropyl, 2-Ethoxycarbonylethyl, 2- oder 3-Ethoxycarbonylpropyl, 2-Dimethylaminocarbonyloxyethyl, 2-Diethylaminocarbonyloxyethyl, 2- oder 3-Dimethylaminocarbonyloxypropyl, 2- oder 3-Diethylaminocarbonyloxypropyl, 2-Methoxycarbonyloxyethyl, 2- oder 3-Methoxycarbonyloxypropyl, 2-Ethoxycarbonyloxyethyl, 2- oder 3-Ethoxycarbonyloxypropyl, 2-Butoxycarbonyloxyethyl, 2- oder 3-Butoxycarbonyloxypropyl, 2-(2-Phenylethoxycarbonyloxy)ethyl, 2- oder 3-(2-Phenylethoxycarbonyloxy)propyl, 2-(2-Ethoxyethoxycarbonyloxy)ethyl, 2- oder 3-(2-Ethoxyethoxycarbonyloxy)propyl, 2-Hydroxyethyl, 2-Hydroxypropyl, 3-Hydroxypropyl, 2-Hydroxybutyl, 4-Hydroxybutyl, 5-Hydroxypentyl, 6-Hydroxyhexyl, 7-Hydroxyheptyl, 8-Hydroxyoctyl, 2-Acryloyloxyethyl, 2-Methacryloyloxyethyl, 2- oder 3-Acryloyloxypropyl, 2- oder 3-Methacryloyloxypropyl, 2- oder 4-Acryloyloxybutyl, 2- oder 4-Methacryloyloxybutyl, 5-Acryloyloxypentyl, 5-Methacryloyloxypentyl, 6-Acryloyloxyhexyl, 6-Methacryloyloxyhexyl, 7-Acryloyloxyheptyl, 7-Methacryloyloxyheptyl, 8-Acryloyloxyoctyl, 8-Methacryloyloxyoctyl, Cyclopentyl, Cyclohexyl, Cycloheptyl, Allyl oder Methallyl.

Reste $R^{18}$ sind z.B. Methylsulfonylamino, Ethylsulfonylamino, Propylsulfonylamino, Isopropylsulfonylamino, Butylsulfonylamino, Mono- oder Dimethylaminosulfonylamino, Mono- oder Diethylaminosulfonylamino, Mono- oder Dipropylaminosulfonylamino, Mono- oder Diisopropylaminosulfonylamino, Mono- oder Dibutylaminosulfonylamino oder (N-Methyl-N-ethylaminosulfonyl)amino.

Reste $R^{23}$ sind weiterhin, wie auch Reste $R^{22}$, z.B. Fluor, Chlor oder Brom.

Reste $R^{23}$ sind weiterhin z.B. Benzyl, 2-Methylbenzyl, 2,4-Dimethylbenzyl, 2-Methoxybenzyl, 2,4-Dimethoxybenzyl, Methylamino, Ethylamino, Propylamino, Isopropylamino, Butylamino, Pentylamino, Hexylamino, Heptylamino, Octylamino oder 2-Ethylhexylamino.

Wenn $R^{20}$ und $R^{21}$ zusammen mit dem sie verbindenden Stickstoffatom einen 5- oder 6-gliedrigen gesättigten heterocyclischen Rest, der gegebenenfalls weitere Heteroatome aufweist, bedeuten, so können dafür z.B. Pyrrolidinyl, Piperidinyl, Morpholinyl, Piperazinyl oder N-($C_1$-$C_4$-Alkyl)piperazinyl in Betracht kommen.

Besonders bevorzugt sind Methinfarbstoffe der Formel Ib, in der $Z^2$ sich von einem Rest der Formel VIIa, VIIf, VIIg oder VIIh ableitet.

Von besonderem Interesse sind Farbstoffe der Formel Ib, in der $Z^2$ sich von einer Komponente aus der Anilinreihe ableitet, dabei insbesondere Reste der Formel VIIa.

Als weiter geeignete Farbstoffe können die erfindungsgemäßen Copolymerisate solche der allgemeinen Formel Ic enthalten

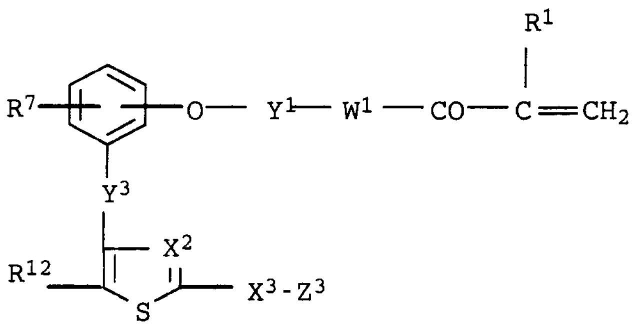

Ic

in der die Substituenten folgende Bedeutung haben:

$R^{12}$ Wasserstoff, $C_1$-$C_{10}$-Alkyl, $C_3$-$C_8$-Cycloalkyl, $C_1$-$C_{10}$-Alkoxy, OH, $NR^8R^9$, CN, $NO_2$, Halogen, CHO,

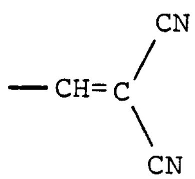

oder

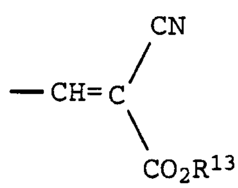

$R^{13}$ Wasserstoff, $C_1$-$C_6$-Alkyl, $C_5$-$C_7$-Cycloalkyl, Phenyl, substituiertes Phenyl oder Benzyl,

$X^2$ N, CH, C-CN, C-$NO_2$ oder C-$CO_2R^{13}$

$X^3$ -N=N-, -CH=N-, -N=CH- oder -CH=CH- und

$Z^3$ ein heteroaromatisches Ringsystem oder

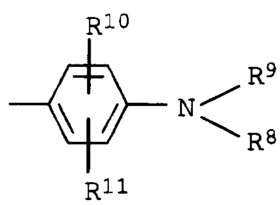

Als Reste $R^8$ und $R^9$ kommen dabei neben Wasserstoff beispielsweise Methyl, Ethyl, Propyl, Isopropyl, Butyl, Isobutyl, sec-Butyl, tert-Butyl, Pentyl, Isopentyl, Neopentyl, tert-Pentyl, Hexyl, Methylpentyl, Cyclopentyl, Cyclohexyl, Cycloheptyl, Phenyl, Methoxyphenyl, Ethoxyphenyl, Propoxyphenyl, Isopropoxyphenyl, Butoxyphenyl, Isobutoxyphenyl, sec-Butoxyphenyl, tert-Butoxyphenyl, Pentoxyphenyl oder Hexoxyphenyl in Betracht.

Weiterhin können $R^8$ und $R^9$ miteinander verbunden sein und zusammen mit dem N-Atom eine Pyrrolidin- oder Piperidingruppe bilden.

Weiterhin können $R^8$ und $R^9$ vernetzungsfähige Gruppen sein wie beispielsweise Acryloyl, Vinyl, Methacryloyl, Oxiranyl, Thiiranyl oder eine Hydroxyalkylgruppe, wobei diese reaktive Gruppen auch über einen bis zu 10 C-Atome

langen Alkylenspacer mit dem Stickstoffatom verbunden sein können.

Als Reste $R^{10}$ und $R^{11}$ kommen neben Wasserstoff beispielsweise Methyl, Ethyl, Propyl, Isopropyl, Butyl, Isobutyl, sec-Butyl, tert-Butyl, Pentyl, Isopentyl, Neopentyl, tert-Pentyl, Hexyl, Cyclopentyl, Cyclohexyl, Cycloheptyl, Cyano, Fluor, Chlor oder $NO_2$ in Betracht.

Als Reste $R^{12}$ kommen neben Wasserstoff beispielsweise Methyl, Ethyl, Propyl, Isopropyl, Butyl, Isobutyl, sec-Butyl, tert-Butyl, Pentyl, sec-Pentyl, tert-Pentyl, Neopentyl, Hexyl, 2-Ethylhexyl oder Octyl in Betracht.

Weitere Reste $R^{12}$ sind beispielsweise Cyclopropyl, Cyclobutyl, Cyclopentyl, Cyclohexyl, Cycloheptyl, Methylcyclopentyl, Methoxy, Ethoxy, Propoxy, Isopropoxy, Butoxy, Isobutoxy, sec-Butoxy, Pentyloxy, Isopentyloxy, Neopentyloxy, tert-Pentyloxy, Hexyloxy, 2-Methylpentoxy, Heptyloxy, Octyloxy, 2-Ethylhexyloxy, Nonyloxy oder Decyloxy.

Weitere Reste $R^{12}$ sind beispielsweise OH, Methylamino, Ethylamino, Propylamino, Isopropylamino, Butylamino, Dimethylamino, Diethylamino, Dipropylamino, Diisopropylamino, Dibutylamino, Cyan, Nitro, Fluor, Chlor, CHO oder Reste

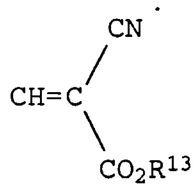

Als Reste $R^{13}$ kommen neben Wasserstoff beispielsweise Methyl, Ethyl, Propyl, Isoproyl, Butyl, Isobutyl, sec-Butyl, tert-Butyl, Pentyl, Isopentyl, Neopentyl, tert-Pentyl, Methylpentyl, Hexyl, Cyclopentyl, Methylcyclopentyl, Cyclohexyl, Cycloheptyl, Phenyl, durch die hiergenannten Alkylgruppen oder entsprechende Alkoxygruppen substituiertes Phenyl oder Benzyl in Betracht.

$Z^3$ kann ein heteroaromatisches Ringsystem sein wie beispielsweise

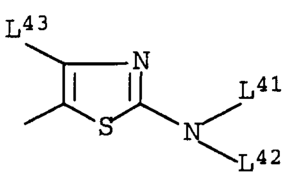

,

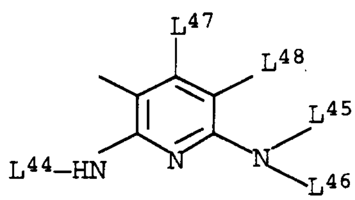

oder

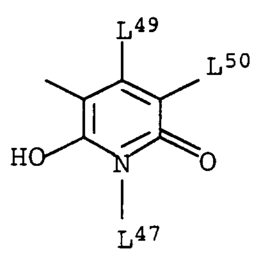

bedeuten, worin

$L^{41}$ und $L^{42}$ gleich oder verschieden sind und jeweils Wasserstoff, gegebenenfalls substituiertes $C_1$-$C_6$-Alkyl, das durch 1 oder 2 Sauerstoffatome in Etherfunktion unterbrochen sein kann, $C_5$-$C_7$-Cycloalkyl oder $C_3$-$C_6$-Alkenyl,

$L^{43}$ Wasserstoff, $C_1$-$C_{12}$-Alkyl, gegebenenfalls substituiertes Phenyl oder Thienyl,

$L^{44}$, $L^{45}$, $L^{46}$ gleich oder verschieden sind und jeweils gegebenenfalls substituiertes $C_1$-$C_{12}$-Alkyl, das durch 1 bis 3 Sauerstoffatome in Etherfunktion unterbrochen sein kann,
$C_5$-$C_7$-Cycloalkyl, gegebenenfalls substituiertes Phenyl, $C_3$-$C_6$-Alkenyl, gegebenenfalls substituiertes Benzoyl, $C_1$-$C_8$-Alkanoyl, $C_1$-$C_6$-Alkylsulfonyl oder gegebenenfalls substituiertes Phenylsulfonyl oder $L^{38}$ und $L^{39}$ zusammen mit dem sie verbindenden Stickstoff einen 5- oder 6-gliedrigen gesättigten heterocyclischen Rest, der gegebenenfalls weitere Heteroatome enthält,

$L^{47}$ Wasserstoff oder $C_1$-$C_6$-Alkyl,

$L^{48}$ Cyano, Carbamoyl oder Acetyl,

$L^{49}$ Wasserstoff oder $C_1$-$C_6$-Alkyl, und

$L^{50}$ Cyano, Carbamoyl oder Acetyl

bedeuten.

Besonders bevorzugt sind erfindungsgemäße Copolymerisate, in denen das Farbstoff -Monomere Ic eine der folgenden Strukturen aufweist:

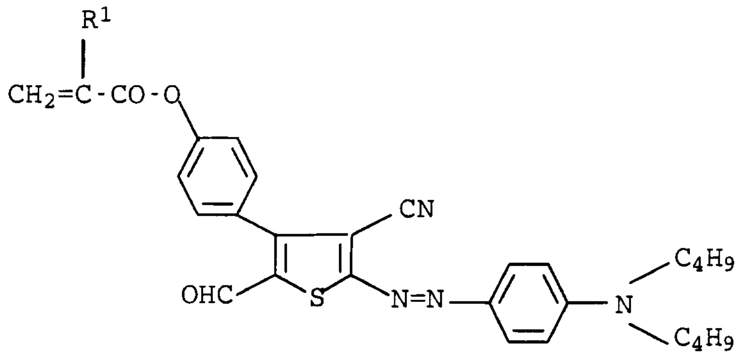

1

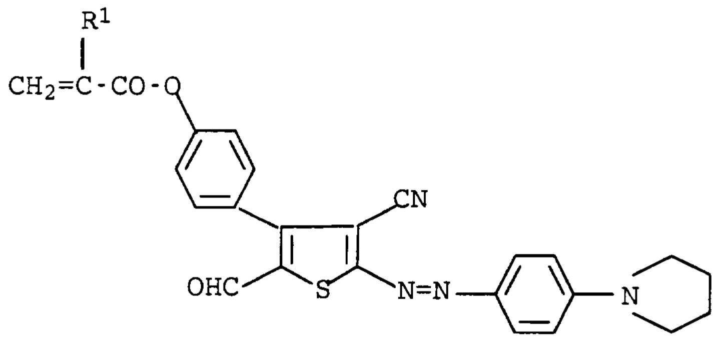

2

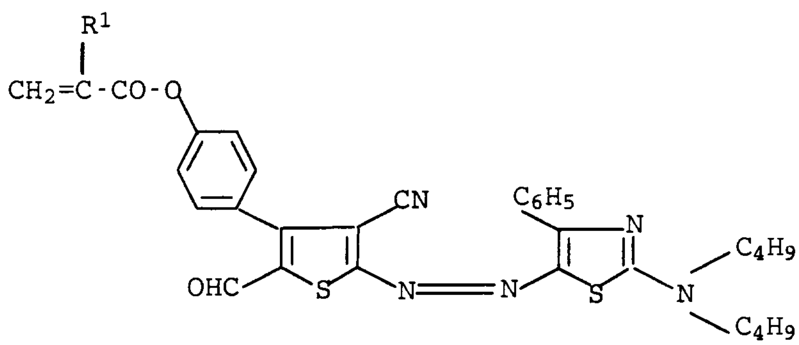

3

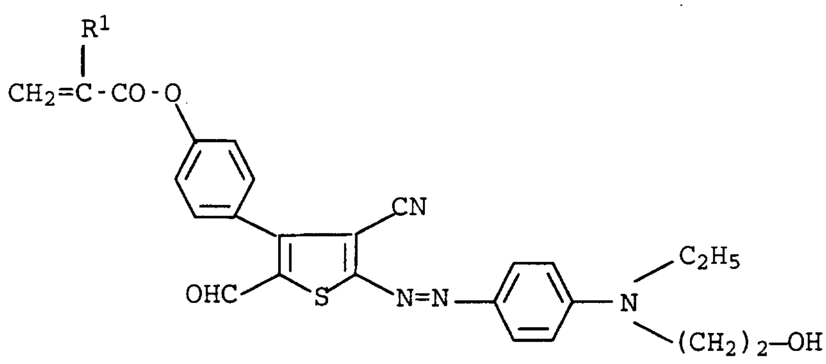

4

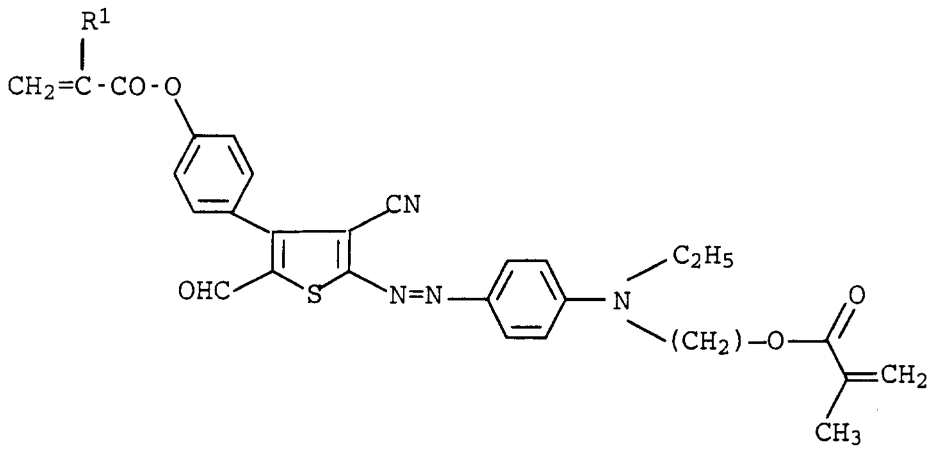

5

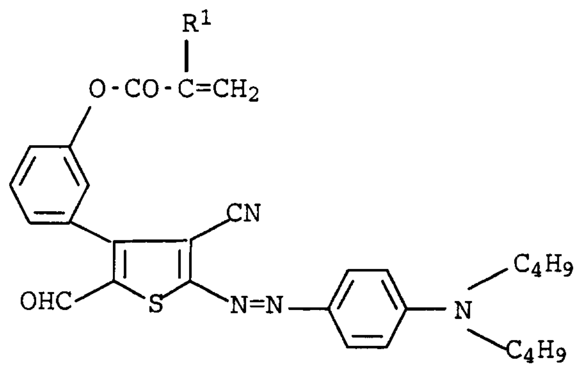

6

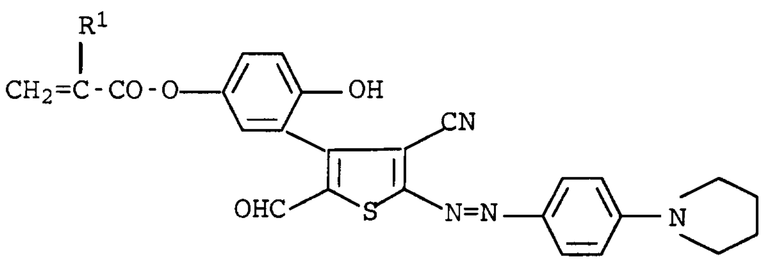

7

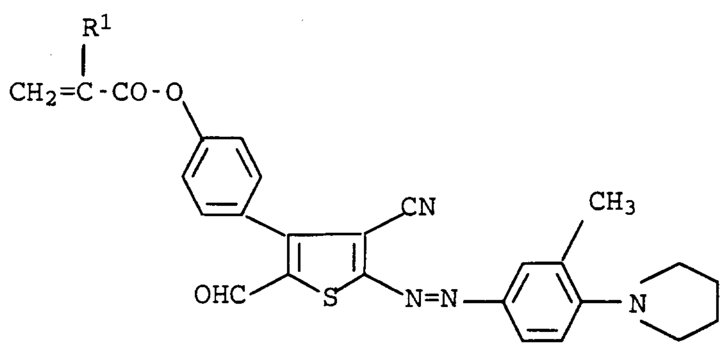

8

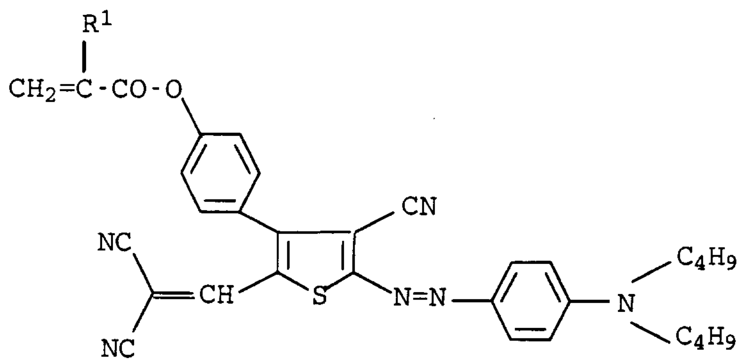

9

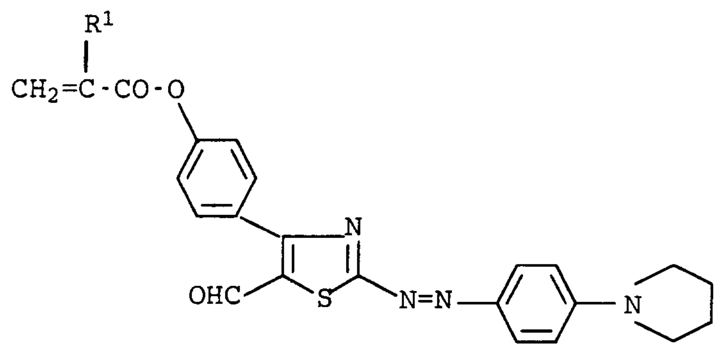

10

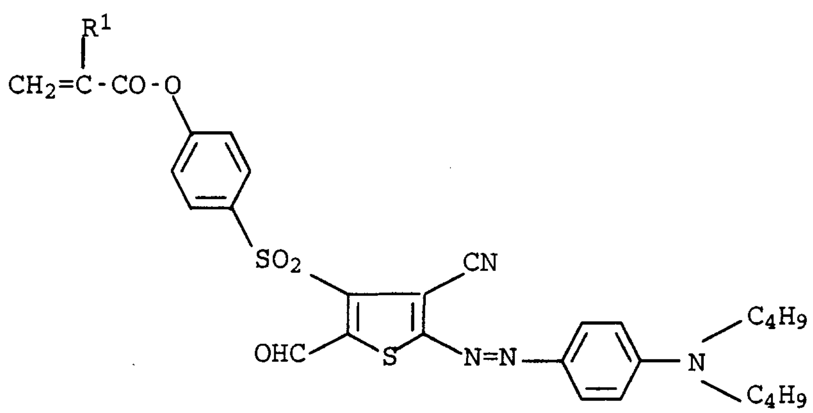

11

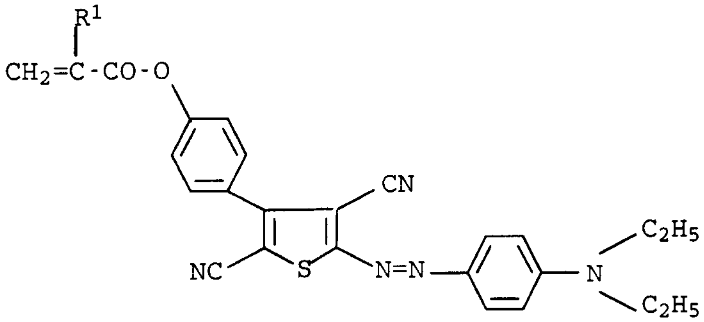

12

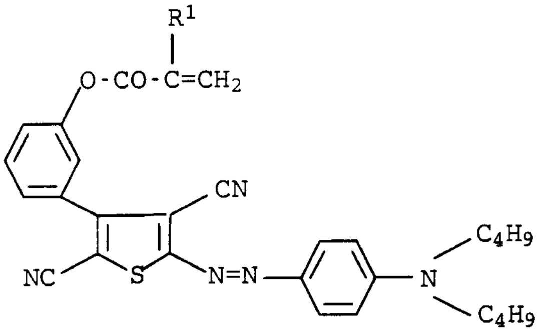

13

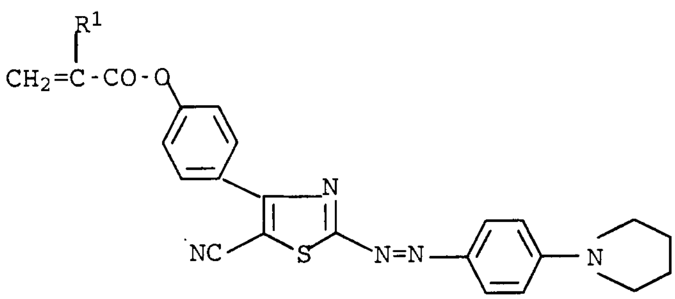

14

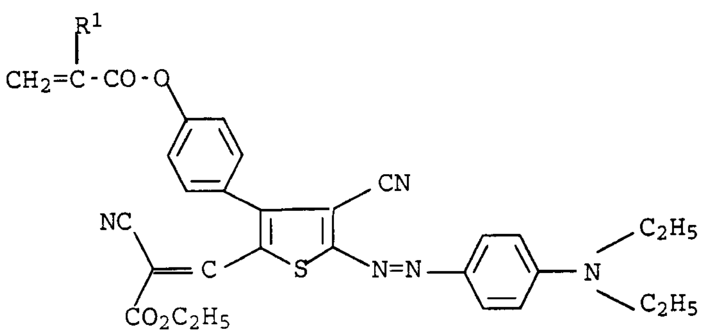

15

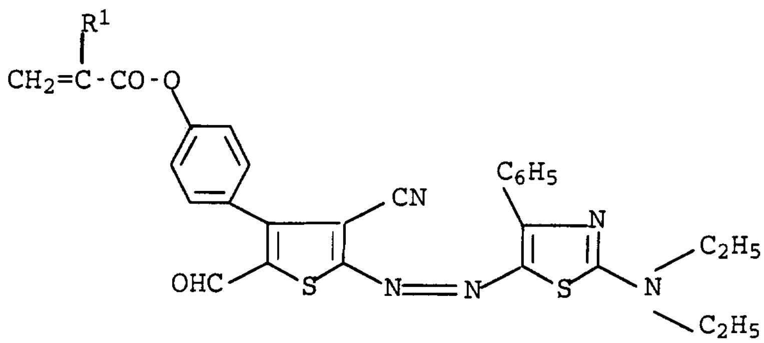

16

Neben den NLO-Farbstoffen ist das Monomere II, welches in der Seitenkette den Adamantylrest tragt, wesentlicher Bestandteil der erfindungsgemäßen Copolymere.

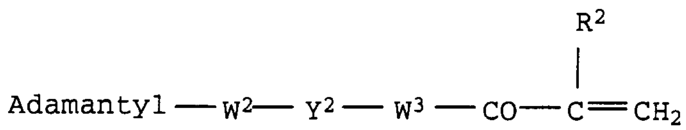

II

Dabei kommen für $Y^2$ die gleichen Gruppen wie für $Y^1$ und für $W^2$ und $W^3$ die gleichen Gruppen wie für $W^1$ in Betracht. Eine bevorzugte Gruppe $W^2$-$Y^2$ ist dabei die chemische Bindung.

Bevorzugte Reste $W^2$ und $W^3$ sind Sauerstoffatome.

Bevorzugte Reste $R^2$ sind Wasserstoff und Methyl.

Neben den Monomeren I und II können die erfindungsgemäßen Polymerisate noch weitere radikalisch polymerisierbare Monomere III enthalten. Bevorzugte Comonomere sind die Monomeren IIIa, IIIb und IIIc:

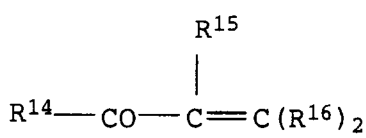

IIIa

$C_6H_5$-CH=$CH_2$ IIIb

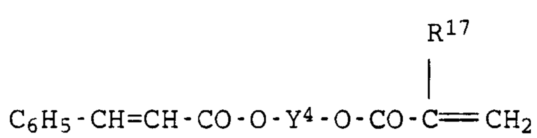

IIIc

enthalten sind, in denen

$R^{14}$ Hydroxy, $C_1$-$C_6$-Alkoxy, trideuteriertes Methoxy, 2,3-Epoxypropoxy, Phenoxy, Amino oder $C_1$-$C_4$-Mono- oder Dialkylamino,

| | |
|---|---|
| $R^{15}$,,$R^{17}$ | ein Rest der Definition von $R^1$, |
| $R^{16}$ | Wasserstoff oder Deuterium, |
| $Y^4$ | ein Rest der Definition von $Y^1$, jedoch keine chemische Bindung |

bedeuten.

Geeignete Reste $R^{14}$ sind beispielsweise Methoxy, Ethoxy, Propoxy, Isopropoxy, Butoxy, Isobutoxy, sec-Butoxy, Pentyloxy, Isopentyloxy, Neopentyloxy, tert.-Pentyloxy, Hexyloxy oder 2-Methylpentyloxy. Weitere geeignete Reste $R^{14}$ sind Methylamino, Ethylamino, Propylamino, Isopropylamino, Butylamino, Dimethylamino, Diethylamino, Dipropylamino, Diisopropylamino, Dibutylamino oder N-Methyl-N-Ethylamino.

Bevorzugt sind Comonomere der Formel IIIa, in der $R^{14}$ gleich $C_1$-$C_4$-Alkoxy, $R^{15}$ gleich Wasserstoff oder Methyl und $R^{16}$ gleich Wasserstoff sind.

Als Brückenglieder $Y^4$ in Formel IIIc sind die gleichen Gruppen geeignet wie für $Y^1$ beschrieben, jedoch keine chemische Bindung.

Als Reste $R^{17}$ eignen sich besonders Wasserstoff und Methyl.

Bevorzugte Polymerisate enthalten die verschiedenen Monomeren in den folgenden Anteilen:

| | |
|---|---|
| Monomer der Formel I: | 4 bis 50 mol-%, besonders bevorzugt 8-25 mol-% |
| Monomer der Formel II | 50 bis 96 mol-%, besonders bevorzugt 75-92 mol-% |
| Monomer der Formel III | 0 bis 30 mol-%, besonders bevorzugt 0-15 mol-%, |

jeweils bezogen auf das Polymerisat.

Das mittlere Molekulargewicht der Polymerisate beträgt bevorzugt 1500 bis 50 000, besonders bevorzugt 2000 bis 25000.

Die Herstellung der neuen Polymerisate kann nach an sich bekannten Methoden, wie sie z.B. in J. Polymer Sci. (loc. cit.) beschrieben sind, erfolgen.

Zweckmäßig setzt man ein chromophorhaltiges Monomeres der Formel I mit einem adamantylenthaltendem Monomeren der Formel II und gegebenenfalls weiterer Monomerer III im oben genannten Molverhältnis in einem inerten Lösungsmittel (z.B. Toluol oder Xylol) in Gegenwart eines Radikalstarters (z.B. Azo-bis-isobutyronitril) um.

Die Farbstoffmonomeren I sind z.T. bekannt. Die Azofarbstoffe können nach den beispielsweise in EP-201 896, DE-A 3 108 077, US-A 4 843 153 und GB-A 1 546 803 sowie in der älteren deutschen Patentanmeldung 44 12 983.1 genannten Methoden erhalten werden. Die Methin- oder Azamethinfarbstoffe können nach den beispielsweise in der EP-A 416 434, EP-A 449 109, EP-A 479 068, EP-A 479 076, EP-A 480 252, DE-A 41 14 456, DE-A 41 34 805 oder DE-A 44 14 882 beschriebenen Methoden erhalten werden.

Die erfindungsgemäßen chromophortragenden Polymerisate eignen sich in vorteilhafter Weise zur Anwendung in nichtlinear optischen Systemen (siehe z.B. Chemistry and Industry, 1. Oktober 1990, Seiten 600 bis 608).

Insbesondere ist hierbei die Eignung der Polymeren in der Nachrichtentechnik, in elektrooptischen Modulatoren (z.B. Mach-Zehnder-Interferometer), in optischen Schaltern, bei der Frequenzmischung oder in Wellenleitern hervorzuheben.

Die Herstellung von Schichten, die die erfindungsgemäßen Polymerisate enthalten, erfolgt dabei in an sich bekannter Weise, z.B. durch Naßbeschichtung (Spincoating) mit einer 5 bis 15 gew.-%igen Lösung des Polymerisats in einem Lösungsmittel (z.B. Tetrachlorethan, Chlorbenzol, Cyclohexanon, Diglyme, N-Methylpyrrolidon).

Bei geeignetem Substitutionsmuster (z.B. Epoxystruktur) können die neuen Polymerisate auch photochemisch, thermisch oder durch Einwirkung von Elektronenstrahlen vernetzt werden.

Die neuen Polymerisate zeichnen sich durch gute Verarbeitbarkeit zu dünnen Schichten, hohe Reinheit, enge Molekulargewichtsverteilung, gute Orientierung im elektrischen Feld, gute Langzeitstabilität, hohe Glasstufen sowie einen hohen elektrooptischen Koeffizienten aus.

Beispiel 1

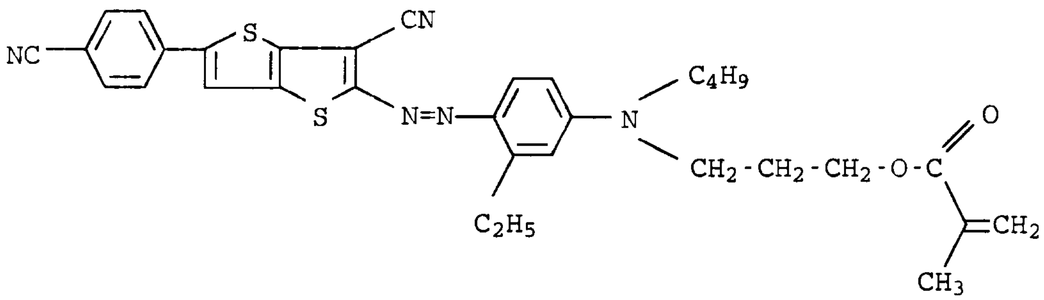

74,2 g (0,264 mol) der Verbindung der Formel

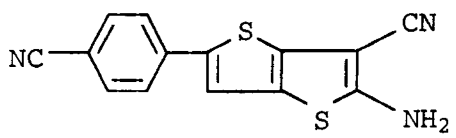

(Die Herstellung der Verbindung ist in der deutschen Patentanmeldung 4 339 712.3 beschrieben) wurden in 475 ml Eisessig/Ameisensäure (1/1; vol/vol) suspendiert. Bei 5°C wurden 99,3 g (0,317 mol) Nitrosylschwefelsäure innerhalb einer Stunde zugetropft. Anschließend wurde die Mischung 5 Stunden bei 5°C gerührt und dann bei 10°C in eine Lösung aus 120 g (0,396 mol) der Verbindung der Formel

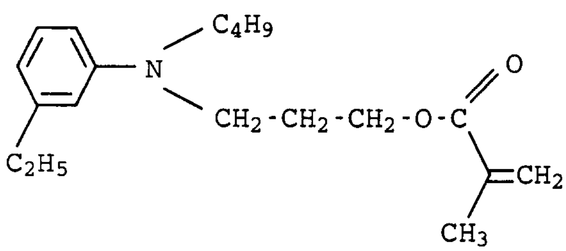

(Herstellung der Verbindung ist der älteren deutschen Patentanmeldung 19521503.6 beschrieben) 6,34 g (0,066 mol) Amidosulfonsäure und 475 ml Eisessig gegeben.

Die Mischung wurde anschließend auf 5,3 l Eiswasser gegossen und über Nacht gerührt. Der entstandene Farbstoff wurde abgesaugt, mit Wasser neutral gewaschen und bei 50°C im Vakuumtrockenschrank getrocknet.

Die Reinigung erfolgte durch Chromatographie an Kieselgel und durch anschließende Umkristallisation aus Toluol.

Schmp.: 162°C
$\lambda_{max}(CH_2CH_2)$: 582 nm
$\mu_g\beta_o$: $1400 \cdot 10^{-80}\ C^2 \cdot m^4/V^2$

Analog Beispiel 1 werden folgende Farbstoffe hergestellt

Beispiel 2

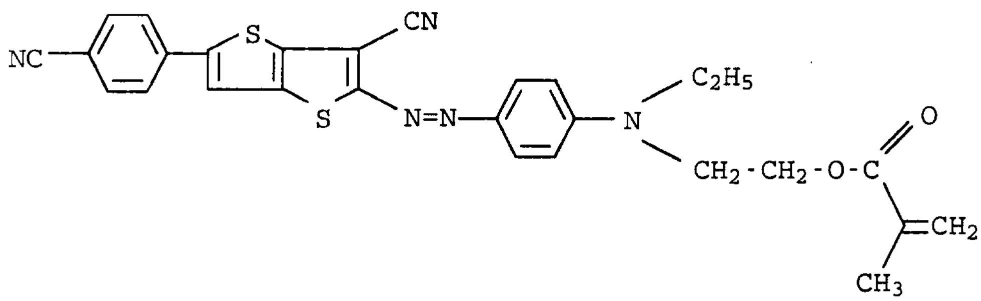

Schmp.: 221-223°C
$\lambda_{max}(CH_2CH_2)$: 562 nm

Beispiel 3

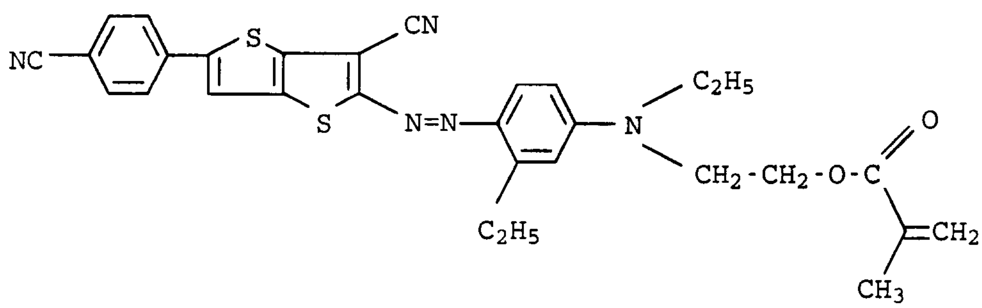

Schmp.: 226-227°C
$\lambda_{max}(CH_2CH_2)$: 572 nm

Beispiel 4

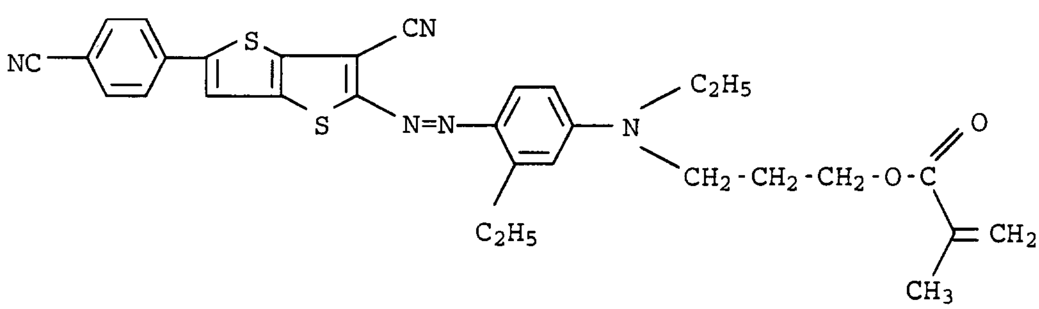

Schmp.: 183-184°C
$\lambda_{max}(CH_2CH_2)$: 580 nm

Beispiel 5

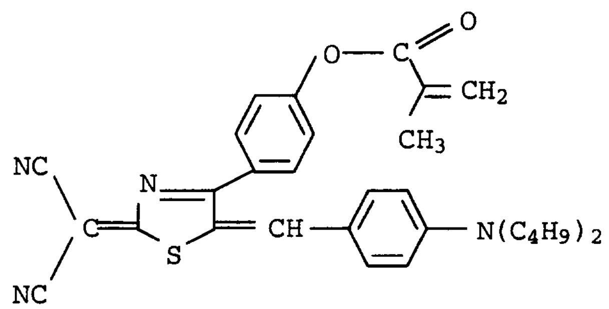

Eine Mischung aus
56,5 g (0,242 mol) der Verbindung der Formel

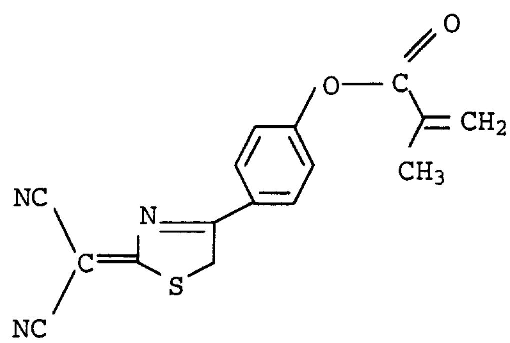

(Die Herstellung der Verbindung ist in der deutschen Patentanmeldung 4 414 882.8 beschrieben.)

74,2 g (0,240 ml) 4-Dibutylaminozimtaldehyd und 383 ml Acetanhydrid wurden für 20 Minuten bei Rückfluß zum Sieden erhitzt. Danach wurde bei Raumtemperatur der Niederschlag abgesaugt und mit Xylol und dann mit Methanol nachgewaschen. Nach dem Trocknen bei 50°C im Vakuumtrockenschrank wurde 59,5 g Rohfarbstoff erhalten, der durch Säulenchromatographe und anschließende Umfällung aus THF/n-Hexan gereinigt wurde.

Schmp.: 188,4°C
$\lambda_{max}(CH_2CH_2)$: 644 nm
$\mu_g\beta_o$: 1600 • $10^{-80}$ $c^2$ • $m^4/V^2$

Beispiel 6

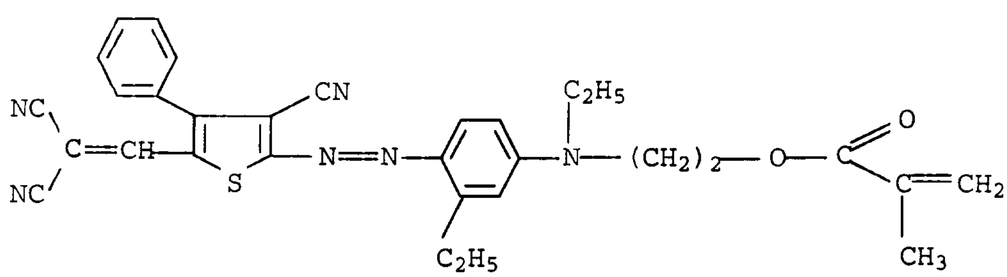

a) Herstellung der Kupplungskomponente:

Zu einer Mischung aus 121 g (1 mol) 3-Ethylanilin und 101 g (1 mol) Triethylamin wurden 102 g (1 mol) Acetanhydrid getropft. Das Gemisch wurde eine Stunde zum Sieden erhitzt. Die Lösung wurde auf Wasser gegeben, mit Essigester extrahiert, die organische Phase wurde mit Wasser gewaschen, getrocknet und unter vermindertem Druck vom Lösungsmittel befreit.

Ausbeute: 160 g der Verbindung 6a

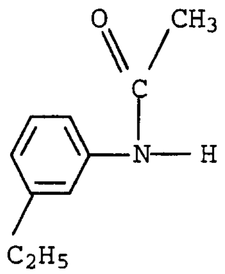

6a

25 g (0,67 mol) Lithiumaluminiumhydrid wurden unter Stickstoff in 100 ml Tetrahydrofuran (THF) suspendiert. Dazu wurden 80 g (0,49 mol) der Verbindung 6a in 350 ml THF bei der Siedetemperatur zugegeben. Anschließend wurde 2 Stunden unter Rückfluß gerührt und dann das Gemisch auf Eiswasser gegeben. Das Gemisch wurde filtriert und weiter wie Verbindung 6a aufgearbeitet.

Ausbeute: 48 g der Verbindung 6b

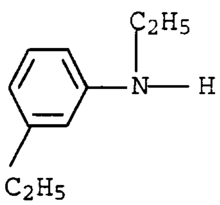

6b

71,5 g (0,48 mol) der Verbindung 6b und 3,3 g (0,024 mol) Zinkchlorid wurden in einem Autoklaven mit Stickstoff gespült und dann unter Druck mit 25 ml Ethylenoxid versetzt. Die Mischung wurde 6 Stunden auf 110°C erhitzt. Die Reinigung erfolgte durch Destillation bei 0,5 mbar.

Ausbeute: 52 g der Verbindung 6c

Siedepunkt: 135-160°C (0,5 mbar)

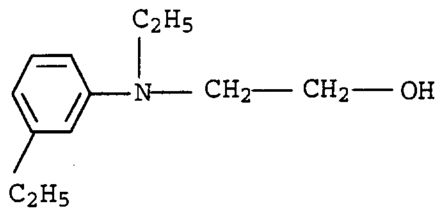

6c

Verbindung 6c wurde in bekannter Weise mit Methacrylsäurechlorid zur Verbindung 6d umgesetzt.

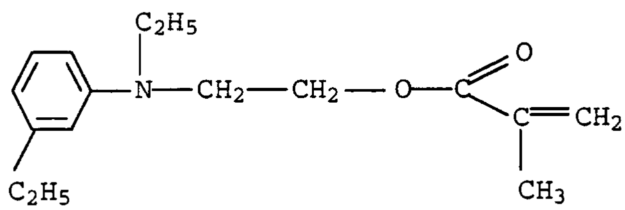

6d

b) Herstellung der Diazokomponente:

Die Herstellung der Diazokomponente 2-Amino-3-cyan-4-phenyl-5-thiophenaldehyd erfolgte analog dem in der DE 28 18 101 beschriebenen Verfahren.

c) Azokupplung

Eine Lösung aus 14,8 g (65 mmol) des Thiophenderivates

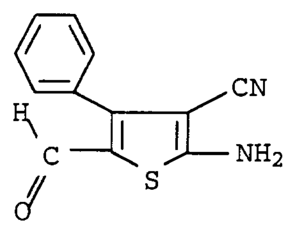

in 115 ml Eisessig/Propionsäure (v/v 7:3) wurde bei 0°C mit 20,5 g (65 mmol) Nitrosylschwefelsäure versetzt. Nach 2 Stunden wurde das Reaktionsgemisch zu einer Lösung von 22,7 g (76 mmol) der Kupplungskomponente

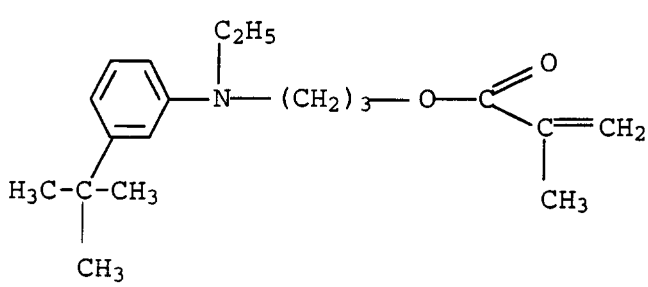

in 100 ml Eisessig gegeben. Nach 30 min wurden 100 g Eis zugegeben, und dann wurde das Reaktionsgemisch noch 15 Stunden bei 20°C gerührt. Das ausgefallene Produkt wurde abfiltriert und mit Wasser gewaschen. Die Reinigung erfolgte durch Säulenchromatographie.

Ausbeute: 6,3 g.
$\lambda_{max}$ ($CH_2Cl_2$):590 nm

d) Umsetzung mit Malonsäuredinitril

6,1 g (11,6 mmol) der unter c) erhaltenen Verbindung in 58 ml THF wurden mit 0,85 g (12,8 mol) Malonsäuredinitril, 2,9 ml Eisessig, 1,45 ml Piperidin und 1,45 g Natriumsulfat versetzt. Das Reaktionsgemisch wurde 1 Stunde zum Sieden erhitzt. Zur Aufarbeitung wurde mit Wasser extrahiert. Die organischen Phasen wurden getrocknet und unter vermindertem Druck vom Lösungsmittel befreit. Die Reinigung erfolgte durch Säulenchromatographie.

Schmp.: 202,5-203,0°C
$\lambda_{max}$($CH_2CH_2$): 650 nm
$\mu_g$-$\beta_o$: $2200 \cdot 10^{-80}$ $c^2 \cdot m^4/V^2$

Beispiel 7

Copolymer aus Adamantylmethacrylat (90 Gew.-%) und der Beispielverbindung 5 (10 Gew.-%)

13,5 g (61,36 mmol) frisch destilliertes Adamantylmethacrylat, 1,5 g (2,86 mmol) Verbindung des Beispiels 5, 170 ml wasser- und sauerstofffreies Chlorbenzol und 0,22 g (1,28 mmol) Azodiisobutyronitril wurden in ein Schlenkrohr gefüllt. Die Lösung wurde sorgfältig entgast und dann 48 Stunden bei 60°C polymerisiert. Zur Aufarbeitung wurde das Polymer in Methanol ausgefällt, isoliert und dann zweimal aus Methylenchlorid/Methanol umgefällt. Nach dem Trocknen bei 50°C im Vakuumschrank wurden 9,2 g Copolymer erhalten.

Gelpermeationschromatographie [a)]

| | |
|---|---|
| GPC: | |
| $\overline{M}_w$: | 31540 |
| $\overline{M}_n$: | 12610 |
| $\overline{M}_w/\overline{M}_n$: | 2,502 |
| Farbstoffgehalt b): | 11,7 % |
| Glasstufe Tg[c)]: | 224°C |

Analog Beispiel 7 wurden die Copolymere 8 bis 18 hergestellt.

a) Die GPC wurde an einem PL-Gel (4-Säulen-Kombination; Porenweite 2x100 nm und 2x100 nm) durchgeführt. Eluent: THF, Kalbrierung mit Polystyrol-Standards

b) Der Farbstoffgehalt wurde über die Elementaranalyse des Copolymeren im Vergleich zur Elementaranalyse des Farbstoffmonomeren über die Stickstoff- und Schwefelwerte bestimmt.

c) Die Glasstufe wurde mit einer Heizrate von 20°C/min bestimmt, die Proben wurden zunächst auf 250°C erwärmt, abgekühlt und dann vermessen.

| Bsp. | Gew.-% Adamantylmeth-acrylat | Gew.-%/Farbstoff | Gew.-%[b] Farbstoff | $\bar{M}_w$[a] | $\bar{M}_n$[a] | $\bar{M}_w/\bar{M}_n$[a] | Tg[c] |
|---|---|---|---|---|---|---|---|
| 8 | 80 | 20/Bsp.5 | 23,6 | 36030 | 20550 | 1,753 | 212°C |
| 9 | 70 | 30/Bsp.5 | 35,5 | 26430 | 15530 | 1,702 | 172°C |
| 10 | 60 | 40/Bsp.5 | 46,3 | 22940 | 13840 | 1,658 | 148°C |
| 11 | 50 | 50/Bsp.5 | 57,7 | 20530 | 12010 | 1,710 | 135°C |
| 12 | 90 | 10/Bsp.1 | 8,3 | 85060 | 21720 | 3,916 | 229°C |
| 13 | 77 | 23/Bsp.1 | 20,0 | 78590 | 21650 | 3,631 | 228°C |
| 14 | 70 | 30/Bsp.1 | 27,1 | 78090 | 21130 | 3,696 | 223°C |
| 15 | 60 | 40/Bsp.1 | 37,8 | 39760 | 15390 | 2,584 | 212°C |
| 16 | 47 | 53/Bsp.1 | 52,5 | 37580 | 14190 | 2,649 | 203°C |
| 17 | 36 | 64/Bsp.1 | 61,8 | 33420 | 13920 | 2,401 | 194°C |
| 18 | 60 | 40/Bsp.6 | 42,3 | 155270 | 14370 | 10,809 | 190°C |

## Patentansprüche

**1.** Adamantylgruppen tragende Copolymere aus

I) 1 bis 99 mol-% eines Monomeren der allgemeinen Formel I

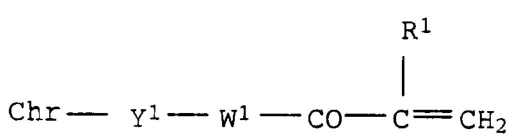

I

II) 1 bis 99 mol-% eines Monomeren der allgemeinen Formel II

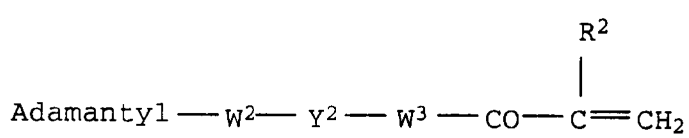

II

und

III) 0 bis 90 mol-% weiterer radikalisch polymerisierbarer Monomerer (III),

wobei die Substituenten und Brückenglieder die folgende Bedeutung haben:

Chr der Rest eines Farbstoffes aus der Methin-, Azamethin-, Azo-, Methylenamino- oder Vinylenreihe, wobei im Falle der Azo-, Methylenamino- und Vinylenfarbstoffreste mindestens einer der aromatischen Ringe des Chromophors mindestens ein Heteroatom enthält,

$R^1$,$R^2$ Wasserstoff, Deuterium, Methyl, trideuteriertes Methyl oder Chlor,

$Y^1$,$Y^2$ $C_2$-$C_{11}$-Alkylen oder durch bis zu 4 Sauerstoffatome in Etherfunktion oder durch nicht benachbarte Imino- oder $C_1$-$C_4$-Alkyliminogruppen unterbrochenes $C_2$-$C_{10}$-Alkylen oder eine chemische Bindung,

$W^1$,$W^2$,$W^3$ Sauerstoff, Imino oder $C_1$-$C_4$-Alkylimino, wobei für den Fall, daß $Y^1$ oder $Y^2$ eine chemische Bindung ist, $W^1$ bzw. $W^2$ entfällt.

**2.** Copolymere nach Anspruch 1, in denen das Monomere der Formel I die Struktur Ia hat,

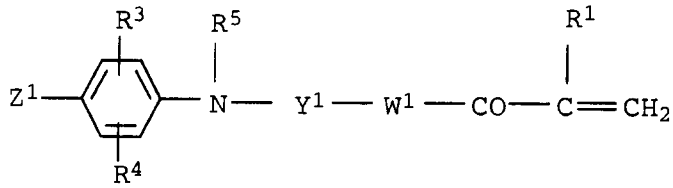

Ia

wobei

$R^3$,$R^4$ Wasserstoff, $C_1$-$C_{12}$-Alkyl, $C_1$-$C_6$-Alkoxy, durch Phenyl oder $C_1$-$C_4$-Alkoxy substituiertes $C_1$-$C_6$-Alkoxy,

$R^5$ Wasserstoff, $C_1$-$C_{12}$-Alkyl, einen Cycloalkylrest mit 5 - 7 C-Atomen im Ring oder $C_3$-$C_4$-Alkenyl und

$Z^1$ eine der folgenden Gruppen
D-N=N-
D-CH=N-
D-N=CH-
D-CH=CH-
G=CH-
G=CH-CH=CH-
G=N-
bedeutet, in denen

D für einen fünfgliedrigen heteroaromatischen Rest mit bis zu drei der Heteroatome Stickstoff, Sauerstoff und/oder Schwefel steht, wobei dieser Rest durch einen Benzol-, Thiophen-, Pyridin- oder Pyrimidinring anelliert sein kann und

G für einen zweiwertigen chinoiden Rest steht, der sich von der Benzol-, Naphthalin-, Pyridin-, Chinolin-, Thiazol-, Benzthiophen-, Triazolopyridin- oder 1,3-Dioxanreihe ableitet.

**3.** Copolymere nach Anspruch 2, in denen D ein Rest der Pyrrol-, Furan-, Thiophen-, Pyrazol-, Imidazol-, Oxazol-, Isoxazol-, Thiazol-, Isothiazol-, Triazol-, Oxdiazol-, Thiadiazol-, Benzofuran-, Benzthiophen-, Benzimidazol-, Benzooxazol-, Benzthiazol-, Benzisothiazol-, Pyridothiophen-, Pyrimidinothiophen-, Thienothiophen- oder Thienothiazolreihe ist.

**4.** Copolymere nach den Ansprüchen 1 bis 3, in denen das Monomere der Formel I folgende Struktur hat

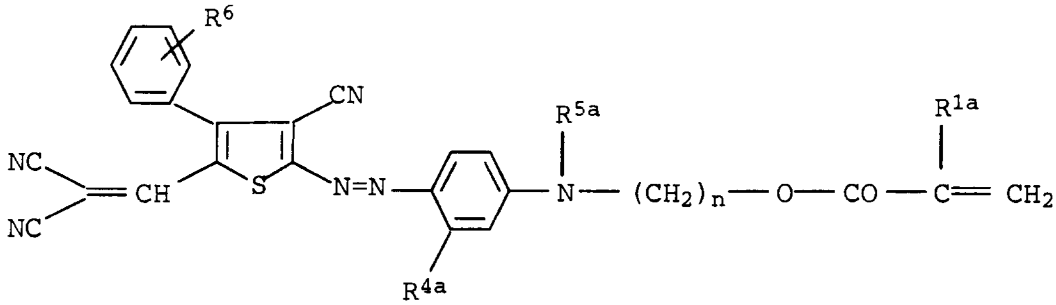

in der

| | |
|---|---|
| $R^6$ | Wasserstoff, $C_1$-$C_6$-Alkyl, $C_1$-$C_6$-Alkoxy oder Hydroxy |
| $R^{4a}$ | Ethyl oder t-Butyl |
| $R^{5a}$ | Ethyl oder Butyl |
| $R^{1a}$ | Wasserstoff oder Methyl und |
| n | 2 bis 6 bedeutet. |

**5.** Copolymere nach Anspruch 2, in denen G einen Rest der Formeln

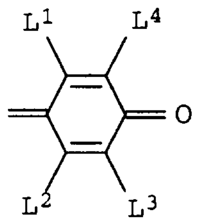
,
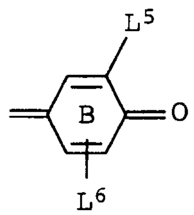
,
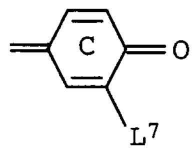
,
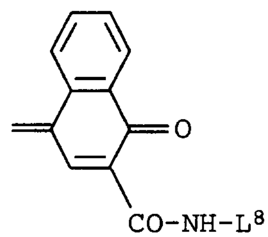
,

(IVa) (IVb) (IVc) (IVd)

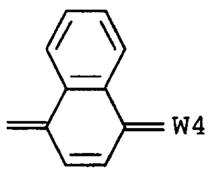
,
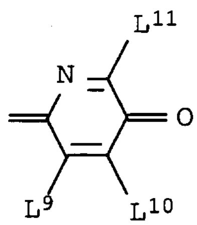
,
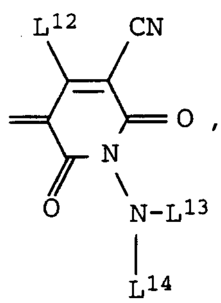
,
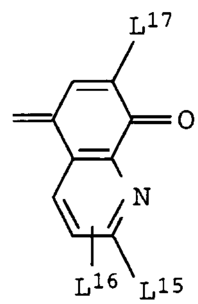
,

(IVe) (IVf) (IVg) (IVh)

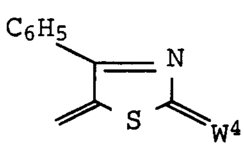
,
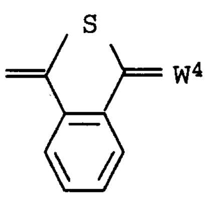
,
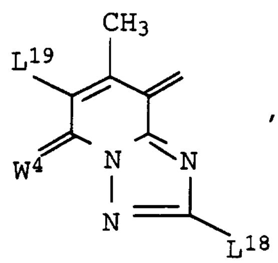
,
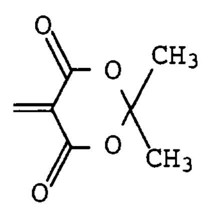
,

(IVi) (VIj) (IVk) (IVl)

bedeutet, worin

die Ringe B und C benzoanelliert sein können und die Substituenten folgende Bedeutung haben:

| | |
|---|---|
| $L^1$, $L^2$ und $L^3$ | unabhängig voneinander jeweils für Wasserstoff, Methyl, Fluor oder Chlor, |
| $L^4$ | Fluor oder Chlor, |
| $L^5$ | Wasserstoff, Fluor, Chlor, Brom, Hydroxy, Nitro, $C_1$-$C_6$-Alkyl, Cyano, $C_1$-$C_4$-Alkylsulfonylamino, $C_1$-$C_6$-Alkoxycarbonyl, $C_1$-$C_4$-Alkylcarbamoyl oder $C_2$-$C_4$-Alkanoylamino, |
| $L^6$ | Wasserstoff, Fluor, Chlor, Cyano, $C_1$-$C_4$-Alkylcarbamoyl, $C_2$-$C_4$-Alkanoylamino, $C_1$-$C_4$-Alkylureido, $C_1$-$C_4$-Alkylsulfamoyl oder $C_1$-$C_6$-Alkoxycarbonyl, |

$L^7$ einen Rest der Formel

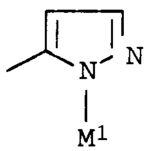 , 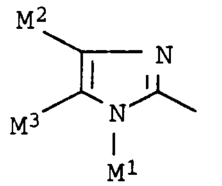 , 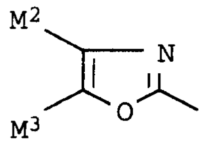 , 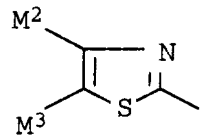 ,

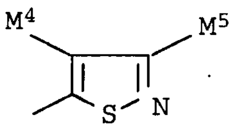 , 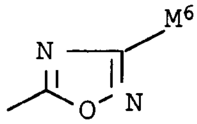 , 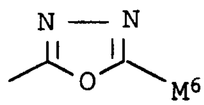 , 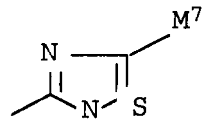

oder 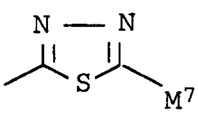 ,

worin die Substituenten folgende Bedeutung haben:

- $M^1$ Wasserstoff, $C_1$-$C_6$-Alkyl, Benzyl, Cyclohexyl, Phenyl oder Tolyl,
- $M^2$ Wasserstoff, Chlor, $C_1$-$C_6$-Alkyl, das durch ein oder zwei Sauerstoffatome in Etherfunktion unterbrochen sein kann, Phenyl, $C_1$-$C_6$-Alkoxy, Cyano oder $C_1$-$C_6$-Alkoxycarbonyl, wobei die Alkylgruppe durch ein oder zwei Sauerstoffatome in Etherfunktion unterbrochen sein kann,
- $M^3$ Wasserstoff, $C_1$-$C_6$-Alkyl, Phenyl, Cyano, Nitro oder $C_1$-$C_6$-Alkoxycarbonyl, wobei die Alkylgruppe durch ein oder zwei Sauerstoffatome in Etherfunktion unterbrochen sein kann, oder $M^2$ und $M^3$ zusammen einen anellierten Benzoring,
- $M^4$ Wasserstoff, Chlor, Cyano, Nitro oder $C_1$-$C_6$-Alkoxycarbonyl, wobei die Alkylgruppe durch ein oder zwei Sauerstoffatome in Etherfunktion unterbrochen sein kann,
- $M^5$ Chlor, $C_1$-$C_6$-Alkyl, das durch ein oder zwei Sauerstoffatome in Etherfunktion unterbrochen sein kann, $C_1$-$C_6$-Alkoxy oder $C_1$-$C_6$-Alkylthio,
- $M^6$ $C_1$-$C_6$-Alkyl und
- $M^7$ Wasserstoff, Chlor, Cyano, Thiocyanato, $C_1$-$C_6$-Alkyl, das durch ein oder zwei Sauerstoffatome in Etherfunktion unterbrochen sein kann, $C_1$-$C_6$-Alkoxy, $C_1$-$C_6$-Alkylthio, 2-($C_1$-$C_2$-Alkoxycarbonyl)ethylthio oder $C_1$-$C_6$-Alkoxycarbonyl, wobei die Alkylgruppe durch ein oder zwei Sauerstoffatome in Etherfunktion unterbrochen sein kann
- $L^8$ einen Rest der Formeln

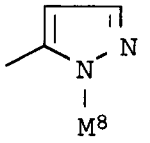 , 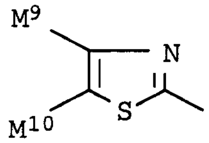 , 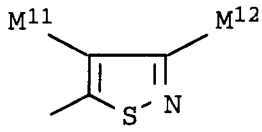 , 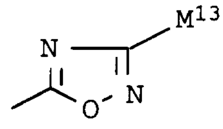 ,

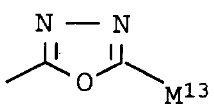 , 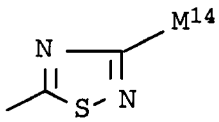 oder 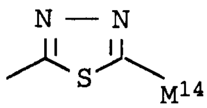 ,

worin die Substituenten folgende Bedeutung haben:

| | |
|---|---|
| $M^8$ | $C_1$-$C_6$-Alkyl oder Cyclohexyl, |
| $M^9$ | Wasserstoff, Chlor, $C_1$-$C_6$-Alkyl, das durch 1 oder 2 Sauerstoffatome in Etherfunktion unterbrochen sein kann, $C_1$-$C_6$-Alkoxy oder $C_1$-$C_6$-Alkoxycarbonyl, wobei die Alkylgruppe durch 1 oder 2 Sauerstoffatome in Etherfunktion unterbrochen sein kann, |
| $M^{10}$ | Wasserstoff, $C_1$-$C_6$-Alkyl, Cyano, Nitro oder $C_1$-$C_6$-Alkoxycarbonyl, wobei die Alkylgruppe durch 1 oder 2 Sauerstoffatome in Etherfunktion unterbrochen sein kann, |
| $M^{11}$ | Wasserstoff, Chlor, Cyano, Nitro oder $C_1$-$C_6$-Alkoxycarbonyl, wobei die Alkylgruppe durch 1 oder 2 Sauerstoffatome in Etherfunktion unterbrochen sein kann, |
| $M^{12}$ | Chlor, $C_1$-$C_6$-Alkyl, das durch 1 oder 2 Sauerstoffatome in Etherfunktion unterbrochen sein kann, $C_1$-$C_6$-Alkoxy oder $C_1$-$C_6$-Alkylthio, |
| $M^{13}$ | $C_1$-$C_6$-Alkyl und |
| $M^{14}$ | Wasserstoff, Chlor, Cyano, Thiocyanato, $C_1$-$C_6$-Alkyl, das durch 1 oder 2 Sauerstoffatome in Etherfunktion unterbrochen sein kann, $C_1$-$C_6$-Alkoxy, $C_1$-$C_6$-Alkylthio, 2-($C_1$-$C_2$-Alkoxycarbonyl)ethylthio oder $C_1$-$C_6$-Alkoxycarbonyl, wobei die Alkylgruppe durch 1 oder 2 Sauerstoffatome in Etherfunktion unterbrochen sein kann, |
| $L^9$ | Wasserstoff, Fluor, Chlor, Methyl oder einen Rest der Formeln<br>$-NH-CO-B^1$, $-NH-CO-OB^1$, $-NH-CO-NB^1B^2$, $-NH-CS-OB^1$, $-NH-CS-NB^1B^2$, $-NH-SO_2-B^1$ oder $-NH-SO_2-NB^1B^2$,<br>worin<br>$B^1$ und $B^2$ $C_1$-$C_4$-Alkyl bezeichnen, |
| $L^{10}$ | Wasserstoff, Fluor oder Chlor oder $L^9$ und $L^{10}$ zusammen mit den Kohlenstoffatomen, an die sie geknüpft sind, einen anellierten Benzoring, |
| $L^{11}$ | einen Rest der Formeln<br>$-CO-OB^1$, $-CO-NHB^1$, $-CO-NH-CO-B^1$, $-NH-CO-B^1$, $-NH-CO-OB^1$, $-NH-CO-NB^1B^2$, $-NH-CS-OB^1$, $-NH-CS-NB^1B^2$, $-NH-SO_2-B^1$ oder $-NH-SO_2-NB^1B^2$, |
| $L^{12}$ | Wasserstoff oder $C_1$-$C_4$-Alkyl, |
| $L^{13}$ und $L^{14}$ | für Wasserstoff, $C_1$-$C_{12}$-Alkyl, $C_5$-$C_7$-Cycloalkyl, Phenyl, Pyridyl, $C_1$-$C_6$-Alkanoyl, $C_1$-$C_6$-Alkoxycarbonyl, $C_1$-$C_6$-Alkylsulfonyl, $C_5$-$C_7$-Cycloalkylsulfonyl, Phenylsulfonyl, Pyridylsulfonyl, Benzoyl, Pyridylcarbonyl oder Thienylcarbonyl oder $L^{13}$ und $L^{14}$ zusammen mit dem sie verbindenden Stickstoffatom einen fünf- oder sechsgliedrigen gesättigten heterocyclischen Rest, der weitere Heteroatome enthalten kann, |
| $L^{15}$ | Wasserstoff oder $C_1$-$C_6$-Alkyl, |
| $L^{16}$ | Wasserstoff, Fluor, Chlor oder Brom, |
| $L^{17}$ | Fluor, Chlor oder Brom, |
| $L^{18}$ | $C_1$-$C_{12}$-Alkyl, das durch ein bis drei Sauerstoffatome in Etherfunktion unterbrochen sein kann, |

Phenyl oder Hydroxy,

$L^{19}$ Cyano, Carbamoyl, Carboxyl oder $C_1$-$C_6$-Alkoxycarbonyl und

$L^{19a}$ $C_1$-$C_6$-Alkyl oder $C_3$-$C_4$-Alkenyl und

$W^4$ Sauerstoff oder einen Rest der Formel

0.

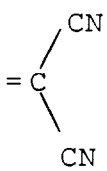 , 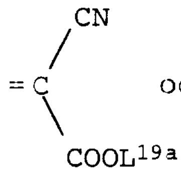 oder 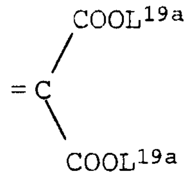

6. Copolymere nach Anspruch 1, in denen das Monomere der Formel I die Struktur Ib hat,

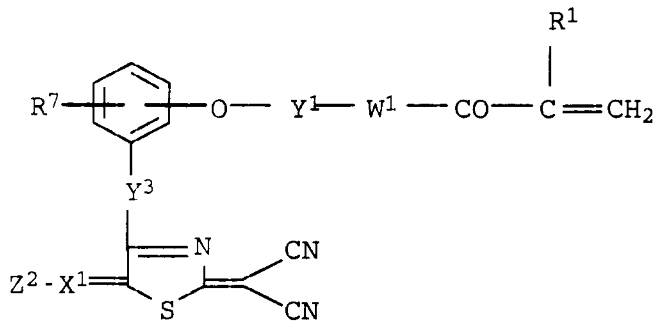

Ib

in der die Substituenten folgende Bedeutung haben:

$Y^3$ eine chemische Bindung, O, S, $SO_2$ oder $NR^5$,

$X^1$ -CH=, -N= oder -CH=CH-CH=

$Z^2$ ein Rest, der sich von einem substituierten oder unsubstituierten Ringsystem aus der Benzol-, Naphthalin-, Indol-, Chinolin-, Thiazol- oder Thiophenreihe ableitet,

$R^7$ Wasserstoff, $C_1$-$C_{10}$-Alkyl, $C_3$-$C_8$-Cycloalkyl, $C_1$-$C_{10}$-Alkoxy, OH, $NR^8R^9$, CN, $NO_2$, Halogen, CHO.

7. Copolymere nach Anspruch 6, in denen

$Z^2$ für einen Rest steht, der sich von einem Ringsystem aus der Anilin-, Aminonaphthalin-, Aminothiazol oder Aminothiophenreihe ableitet.

8. Copolymere nach Anspruch 6 oder 7, in denen das Monomere Ib folgende Struktur hat,

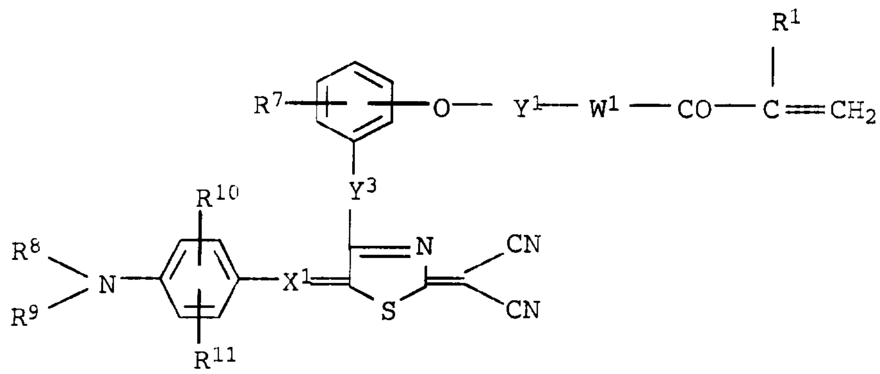

in der

| | |
|---|---|
| $R^8$,$R^9$ | Wasserstoff, $C_1$-$C_6$-Alkyl, $C_5$-$C_7$-Cycloalkyl,eine reaktive Gruppe, über die eine Vernetzung herbeigeführt werden kann, Phenyl oder $C_1$-$C_6$-alkoxysubstituiertes Phenyl, wobei die Reste $R^8$ und $R^9$ auch zu einem Ring geschlossen sein können und |
| $R^{10}$,$R^{11}$ | Wasserstoff, $C_1$-$C_6$-Alkyl, $C_5$-$C_7$-Cycloalkyl, Cyano, Halogen oder $NO_2$ bedeuten. |

**9.** Copolymere nach Anspruch 1, in denen das Monomere der Formel I die Struktur Ic hat,

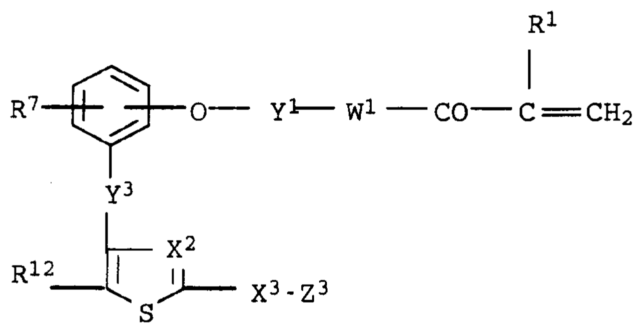

Ic

in der die Substituenten folgende Bedeutung haben:

$R^{12}$ Wasserstoff, $C_1$-$C_{10}$-Alkyl, $C_3$-$C_8$-Cycloalkyl, $C_1$-$C_{10}$-Alkoxy, OH, $NR^8R^9$, CN, $NO_2$, Halogen, CHO,

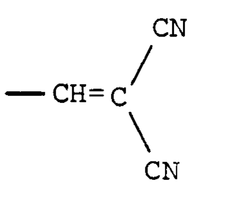

oder

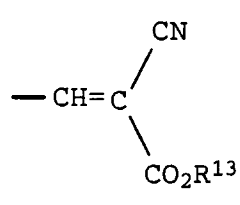

$R^{13}$ Wasserstoff, $C_1$-$C_6$-Alkyl, $C_5$-$C_7$-Cycloalkyl, Phenyl, substituiertes Phenyl oder Benzyl,

$X^2$ N, CH, C-CN, $C$-$NO_2$ oder $C$-$CO_2R^{13}$

$X^3$ -N=N-, -CH=N-, -N=CH- oder -CH=CH- und

$Z^3$ ein heteroaromatisches Ringsystem oder

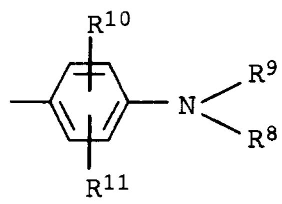

**10.** Copolymere nach Anspruch 9, in denen

$Y^3$ eine chemische Bindung bedeutet.

**11.** Copolymere nach Anspruch 1, in denen als weitere Monomere III eine oder mehrere Verbindungen IIIa, IIIb oder IIIc

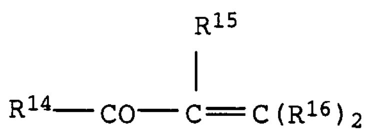

IIIa

$C_6H_5$-CH=$CH_2$ IIIb

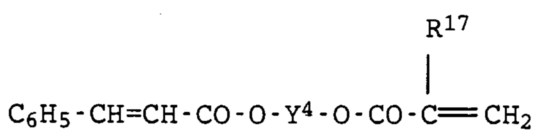

IIIc

enthalten sind, in denen

| | |
|---|---|
| $R^{14}$ | Hydroxy, $C_1$-$C_6$-Alkoxy, trideuteriertes Methoxy, 2,3-Epoxypropoxy, Phenoxy, Amino oder $C_1$-$C_4$-Mono- oder Dialkylamino, |
| $R^{15}$,$R^{17}$ | einen Rest der Definition von $R^1$ |
| $R^{16}$ | Wasserstoff oder Deuterium, |
| $Y^4$ | ein Rest der Definition von $Y^1$, jedoch keine chemische Bindung |

bedeuten.

**12.** Verwendung der Copolymerisate gemäß Anspruch 1 für die Zwecke der nichtlinearen Optik.

**13.** Verfahren zur Herstellung der Copolymeren gemäß Anspruch 1, dadurch gekennzeichnet, daß die Monomeren I, II und III in den angegebenen Mengenverhältnissen in einem Lösungsmittel gelöst und mit Hilfe eines Radikalbildners zur Polymerisation gebracht werden.

Europäisches Patentamt

# EUROPÄISCHER RECHERCHENBERICHT

Nummer der Anmeldung
EP 96 11 0715

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
| X | NONLINEAR OPTICAL PROPERTIES OF ORGANIC MATERIALS VIII, SAN DIEGO, CA, USA, 11-13 JULY 1995, Bd. 2527, ISSN 0277-786X, PROCEEDINGS OF THE SPIE - THE INTERNATIONAL SOCIETY FOR OPTICAL ENGINEERING, 1995, SPIE-INT. SOC. OPT. ENG, USA, Seiten 92-104, XP000605996 ECKL M ET AL: "Nonlinear optical active polymethacrylates" * Seite 98 - Seite 103 * --- | 1-3,9, 12,13 | C08F220/34 C08F220/18 G02F1/35 |
| D,A | EP-A-0 590 421 (BASF AKTIENGESELLSCHAFT) * Ansprüche; Beispiele * --- | 1,12,13 | |
| D,A | EP-A-0 535 490 (BASF AKTIENGESELLSCHAFT) * das ganze Dokument * --- | 1-4,9, 11-13 | |
| D,A | EP-A-0 572 898 (BASF AKTIENGESELLSCHAFT) * das ganze Dokument * ----- | 1,2,5, 11-13 | RECHERCHIERTE SACHGEBIETE (Int.Cl.6) |
| | | | C08F G02F |

Der vorliegende Recherchenbericht wurde für alle Patentansprüche erstellt

| Recherchenort | Abschlußdatum der Recherche | Prüfer |
|---|---|---|
| DEN HAAG | 28.Oktober 1996 | Puetz, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)